# EUROPEAN PATENT APPLICATION

(11) **EP 4 261 207 A1**
(43) Date of publication of application: **18.10.2023**
(21) Application number: 21903641.5
(22) Date of filing: 09.11.2021
(51) Int. Cl.: C07D 215/04, C07D 405/12, C07D 409/12, C07D 401/04, H01L 51/00, H01L 51/50

(54) **HETEROCYCLIC COMPOUND, ORGANIC LIGHT-EMITTING DEVICE COMPRISING SAME, MANUFACTURING METHOD THEREFOR, AND COMPOSITION FOR ORGANIC LAYER**

(30) Priority: 10.12.2020 KR 20200172276
(71) Applicant: LT Materials Co., Ltd., Yongin-si, Gyeonggi-do 17118 (KR)
(72) Inventor: KWON, Su Jin, Yongin-si, Gyeonggi-do 17118 (KR); LEE, Sol, Yongin-si, Gyeonggi-do 17118 (KR); MO, Jun Tae, Yongin-si, Gyeonggi-do 17118 (KR); KIM, Dong Jun, Yongin-si, Gyeonggi-do 17118 (KR)
(74) Representative: Goddar, Heinz J.
(86) International application number: PCT/KR2021/016210
(87) International publication number: WO 2022/124594

(57) **Abstract**

The present specification relates to a heterocyclic compound represented by Formula 1, an organic light-emitting device comprising the same, a manufacturing method thereof, and a composition for an organic layer thereof.

## Description

### [Technical Field]

This application claims the benefit of priority based on Korean Patent Application No. 10-2020-0172276 filed on December 10, 2020, the entire contents of which are incorporated herein as part of the present specification.

The present invention relates to a heterocyclic compound, an organic light-emitting device comprising the same, a manufacturing method thereof, and a composition for an organic layer thereof.

### [Background Art]

An organic light-emitting device is a kind of self-emitting display device, and has the advantages that the viewing angle is wide, the contrast is excellent, and the response speed is fast.

The organic light-emitting device has a structure in which an organic thin film is disposed between two electrodes. When a voltage is applied to the organic light-emitting device having such a structure, electrons and holes injected from the two electrodes combine in the organic thin film to form a pair, and then emit light while disappearing. The organic thin film may be composed of a single layer or multiple layers, if necessary.

The material for the organic thin film may have a light-emitting function, if necessary. For example, as a material for the organic thin film, a compound capable of constituting the light-emitting layer by itself may be used, or a compound capable of serving as a host or dopant of the host-dopant-based light-emitting layer may be used. In addition, as a material for the organic thin film, a compound capable of performing the roles of hole injection, hole transport, electron blocking, hole blocking, electron transport, electron injection, and the like may be used.

In order to improve the performance, lifetime, or efficiency of the organic light-emitting device, there is a continuous demand for the development of materials for the organic thin film.

### [Prior Art References]

### [Patent Documents]

(Patent Literature 1) U.S. Patent No. US 4,356,429

### [Disclosure]

### [Technical Problem]

It is an object of the present invention to provide a heterocyclic compound, an organic light-emitting device comprising the same, a manufacturing method thereof, and a composition for an organic layer thereof.

### [Technical Solution]

The present invention provides a heterocyclic compound represented by following Formula 1: 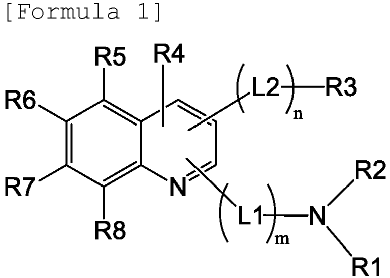 wherein,
L1 and L2 are the same as or different from each other and are each independently a single bond; a substituted or unsubstituted C6 to C60 arylene group; or a substituted or unsubstituted C2 to C60 heteroarylene group,
each of m and n is an integer from 0 to 5, with the proviso that when m is 2 or more, L1 is the same as or different from each other, and when n is 2 or more, L2 is the same as or different from each other,
R1 to R3 are the same as or different from each other and are each independently a substituted or unsubstituted C6 to C60 aryl group or a substituted or unsubstituted C2 to C60 heteroaryl group, and
R4 to R8 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102;-SiR101R102R103; and -NR101R102, or two or more groups adjacent to each other combine with each other to form a substituted or unsubstituted C6 to C60 aromatic hydrocarbon ring or a substituted or unsubstituted C2 to C60 heterocycle, wherein R101, R102, and R103 are the same as or different from each other, and are each independently a substituted or unsubstituted C1 to C60 alkyl group; a substituted or unsubstituted C6 to C60 aryl group; or a substituted or unsubstituted C2 to C60 heteroaryl group.

In addition, the present invention provides an organic light-emitting device comprising a first electrode; a second electrode provided to face the first electrode; and one or more organic layers provided between the first electrode and the second electrode, and wherein at least one of the one or more organic layers comprises the heterocyclic compound represented by Formula 1.

In addition, the present invention provides the organic light-emitting device wherein the organic layer further comprises a heterocyclic compound represented by following Formula 2: 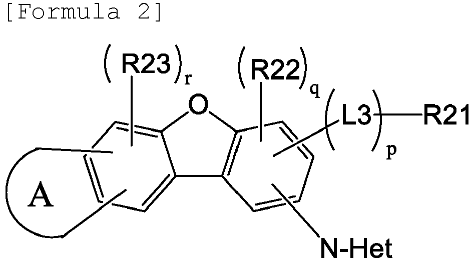 wherein,
N-Het is a substituted or unsubstituted, C2 to C60 monocyclic or polycyclic heterocyclic group containing one or more N,
L3 is a single bond; a substituted or unsubstituted C6 to C60 arylene group; or a substituted or unsubstituted C2 to C60 heteroarylene group, and p is an integer from 0 to 3, with the proviso that when p is 2 or more, L3 is the same as or different from each other,
A is a substituted or unsubstituted C6 to C60 aryl ring or a substituted or unsubstituted C2 to C60 heteroaryl ring,
R21 to R23 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R201R202;-SiR201R202R203; and -NR201R202, or two or more groups adjacent to each other combine with each other to form a substituted or unsubstituted C6 to C60 aromatic hydrocarbon ring or a substituted or unsubstituted C2 to C60 heterocycle, wherein R201, R202, and R203 are the same as or different from each other and are each independently a substituted or unsubstituted C1 to C60 alkyl group; a substituted or unsubstituted C6 to C60 aryl group; or a substituted or unsubstituted C2 to C60 heteroaryl group, and
each of q and r is an integer from 0 to 2, with the proviso that when q is 2, R22 is the same as or different from each other, and when r is 2, R23 is the same as or different from each other.

In addition, the present invention provides a composition for an organic layer of the organic light-emitting device comprising the heterocyclic compound represented by Formula 1 and the heterocyclic compound represented by Formula 2.

In addition, the present invention provides a method of manufacturing an organic light-emitting device, comprising the steps of: preparing a substrate; forming a first electrode on the substrate; forming one or more organic layers on the first electrode; and forming a second electrode on the organic layer, and wherein the step of forming the organic layers comprises a step of forming one or more organic layers using the composition for an organic layer of the organic light-emitting device.

### [Advantageous Effects]

The compounds described in the specification may be used as a material for an organic layer of an organic light-emitting device. The compound may serve as a material for a hole injection layer, a material for a hole transport layer, a material for a light-emitting layer, a material for an electron transport layer, a material for an electron injection layer, and the like in an organic light-emitting device. In particular, the compound may be used as a material for a light-emitting layer of an organic light-emitting device.

Specifically, the compound may be used alone as a light-emitting material, and may be used as a host material or a dopant material of the light-emitting layer. When the compound represented by Formula 1 is used for the organic layer, it is possible to lower the driving voltage, improve the luminous efficiency, and improve the lifetime properties, of the organic light-emitting device.

In particular, in the heterocyclic compound represented by Formula 1 of the present invention, the LUMO orbital is delocalized to improve the stability and mobility of electrons, thereby exhibiting an effect of improving the lifetime of the organic light-emitting device.

In addition, the heterocyclic compound represented by Formula 1 of the present invention has a high triplet energy level (T₁ level), thereby preventing retrograde energy transfer from the dopant to the host, and exhibiting an effect of well preserving triplet excitons in the light-emitting layer.

In addition, the heterocyclic compound represented by Formula 1 above of the present invention facilitates intramolecular charge transfer and reduces the energy gap between the singlet energy level (S₁) and the triplet energy level (T₁), thereby exhibiting an effect of well preserving excitons.

### [Description of Drawings]

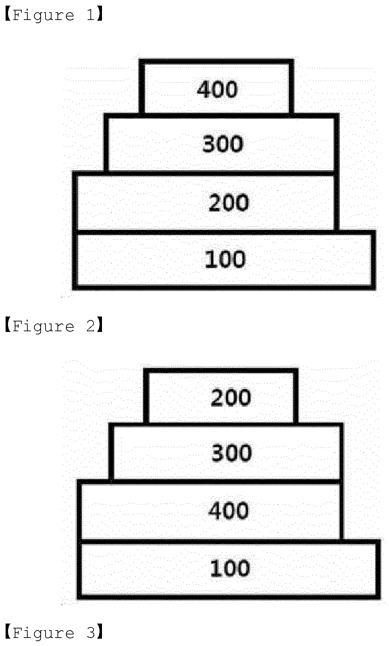

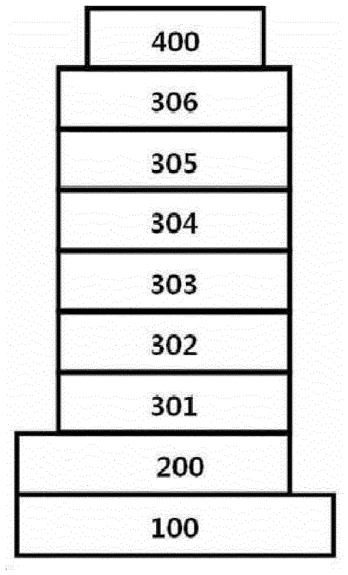

Figs. 1 to 3 are views schematically showing a stacked structure of an organic light-emitting device according to one embodiment of the present invention, respectively.

### [Best Mode]

Hereinafter, the present application will be described in detail.

In the present specification, the term "substituted" means that a hydrogen atom bonded to a carbon atom of a compound is replaced with another substituent, and the position to be substituted is not limited as long as it is the position at which a hydrogen atom is substituted, that is, the position at which it may be substituted with a substituent. When substituted with two or more substituents, the two or more substituents may be the same as or different from each other.

In the present specification, the term "substituted or unsubstituted" means that it is unsubstituted or substituted with one or more substituents selected from the group consisting of deuterium; halogen; cyano; C1 to C60 linear or branched alkyl; C2 to C60 linear or branched alkenyl; C2 to C60 linear or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; - SiRR'R"; -P(=O)RR'; C1 to C20 alkylamine; C6 to C60 monocyclic or polycyclic arylamine; and C2 to C60 monocyclic or polycyclic heteroarylamine, or that it is unsubstituted or substituted with a substituent in which two or more substituents selected from the above-exemplified substituents are connected to each other.

In the present specification, the halogen may be fluorine, chlorine, bromine, or iodine.

In the present specification, the alkyl group includes a linear or branched chain having 1 to 60 carbon atoms, and may be further substituted with another substituent. The number of carbon atoms in the alkyl group may be 1 to 60, specifically 1 to 40, more specifically 1 to 20. Specific examples include, but are not limited to, a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like.

In the present specification, the alkenyl group includes a linear or branched chain having 2 to 60 carbon atoms, and may be further substituted with another substituent. The number of carbon atoms in the alkenyl group may be 2 to 60, specifically 2 to 40, more specifically 2 to 20. Specific examples include, but are not limited to, a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like.

In the present specification, the alkynyl group includes a linear or branched chain having 2 to 60 carbon atoms, and may be further substituted with another substituent. The number of carbon atoms in the alkynyl group may be 2 to 60, specifically 2 to 40, more specifically 2 to 20.

In the present specification, the alkoxy group may be a linear chain, a branched chain, or a cyclic chain. Although the number of carbon atoms in the alkoxy group is not particularly limited, it is preferable that the number of carbon atoms is 1-20. Specifically, it may include, but is not limited to, methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like.

In the present specification, the cycloalkyl group includes a monocyclic or polycyclic ring having 3 to 60 carbon atoms, and may be further substituted with another substituent. In this case, the polycyclic ring refers to a group in which a cycloalkyl group is directly connected or condensed with another cyclic group. In this case, the another cyclic group may be a cycloalkyl group, but may be a different type of cyclic group, for example, a heterocycloalkyl group, an aryl group, a heteroaryl group, or the like. The number of carbon atoms in the cycloalkyl group may be 3 to 60, specifically 3 to 40, more specifically 5 to 20. Specifically, it includes, but is not limited to, a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, 4-tert-butylcyclohexyl group, a cycloheptyl group, a cyclooctyl group, and the like.

In the present invention, the heterocycloalkyl group includes a monocyclic or polycyclic ring containing O, S, Se, N or Si as a heteroatom and having 2 to 60 carbon atoms, and may be further substituted with another substituent. In this case, the polycyclic group refers to a group in which a heterocycloalkyl group is directly connected or condensed with another cyclic group. In this case, the another cyclic group may be a heterocycloalkyl group, but may be a different type of cyclic group, for example, a cycloalkyl group, an aryl group, a heteroaryl group, or the like. The number of carbon atoms in the heterocycloalkyl group may be 2 to 60, specifically 2 to 40, more specifically 3 to 20.

In the present specification, the aryl group includes a monocyclic or polycyclic ring having 6 to 60 carbon atoms, and may be further substituted with another substituent. In this case, the polycyclic group refers to a group in which an aryl group is directly connected or condensed with another cyclic group. In this case, the another cyclic group may be an aryl group, but may be a different type of cyclic group, for example, a cycloalkyl group, a heterocycloalkyl group, a heteroaryl group, or the like. The aryl group includes a spiro group. The number of carbon atoms in the aryl group may be 6 to 60, specifically 6 to 40, more specifically 6 to 25. Specific examples of the aryl group may include, but are not limited to, a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, tetracenyl group, a pentacenyl group, a fluorenyl group, an indenyl group, an acenaphthylenyl group, a benzofluorenyl group, a spirobifluorenyl group, a 2,3-dihydro-1H-indenyl group, condensed cyclic groups thereof, and the like.

In the present specification, the phosphine oxide group is represented by -P(=O)R₁₀₁R₁₀₂, wherein R₁₀₁ and R₁₀₂ may be the same as or different from each other and may be each independently a substituent consisting of at least one of hydrogen; deuterium; a halogen group; an alkyl group; an alkenyl group; an alkoxy group; a cycloalkyl group; an aryl group; and a heterocyclic group. Specifically, it may be substituted with an aryl group, wherein the aryl group may be as exemplified above. For example, the phosphine oxide group includes, but is not limited to, a diphenyl phosphine oxide group, a dinaphthyl phosphine oxide group, and the like.

In the present specification, the silyl group may be a substituent containing Si, in which the Si atom is directly connected as a radical, and may be represented by -SiR₁₀₄R₁₀₅R₁₀₆, wherein R₁₀₄ to R₁₀₆ may be the same as or different from each other and may be each independently a substituent consisting of at least one of hydrogen; deuterium; a halogen group; an alkyl group; an alkenyl group; an alkoxy group; a cycloalkyl group; an aryl group; and a heterocyclic group. Specific examples of the silyl group includes, but is not limited to, a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like.

In the present specification, the fluorenyl group may be substituted, and adjacent substituents may be bonded to each other to form a ring.
When the fluorenyl group is substituted, it may be, but is not limited to, 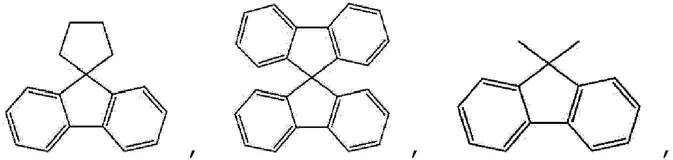 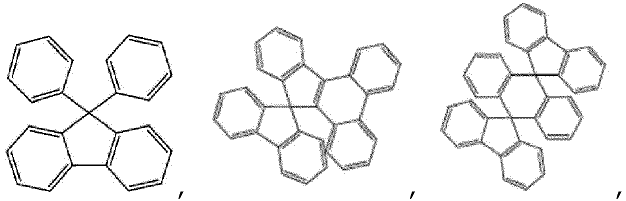 or the like.

In the present specification, the heteroaryl group includes a monocyclic or polycyclic ring containing S, O, Se, N or Si as a heteroatom and having 2 to 60 carbon atoms, and may be further substituted with another substituent. In this case, the polycyclic group refers to a group in which a heteroaryl group is directly connected or condensed with another cyclic group. In this case, the another cyclic group may be a heteroaryl group, but may be a different type of cyclic group, for example, a cycloalkyl group, a heterocycloalkyl group, an aryl group, or the like. The number of carbon atoms in the heteroaryl group may be 2 to 60, specifically 2 to 40, more specifically 3 to 25. Specific examples of the heteroaryl group may include, but are not limited to, a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a quinozolylyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilol group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiazinyl group, a phthalazinyl group, a naphthylidinyl group, a phenanthrolinyl group, a benzo[c][1,2,5]thiadiazolyl group, 5,10-dihydrodibenzo[b,e] [1,4]azasilinyl, a pyrazolo[1,5-c]quinazolinyl group, a pyrido[1,2-b]indazolyl group, a pyrido[1,2-a]imidazo[1,2-e]indolinyl group, a 5, 11-dihydroindeno [1, 2-b] carbazolyl group, and the like.

In the present specification, the amine group may be selected from the group consisting of a monoalkylamine group, a monoarylamine group, a monoheteroarylamine group, -NH₂, a dialkylamine group, a diarylamine group, a diheteroarylamine group, an alkylarylamine group, an alkylheteroarylamine group, and an arylheteroarylamine group; and the number of carbon atoms is not particularly limited, but is preferably 1 to 30. Specific examples of the amine group include, but are not limited to, a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, and the like.

In the present specification, the arylene group refers to a group having two bonding positions on the aryl group, that is, a divalent group. The above description of the aryl group may be applied, except that each of them is a divalent group. In addition, the heteroarylene group refers to a group having two bonding positions on the heteroaryl group, that is, a divalent group. The above description of the heteroaryl group may be applied, except that each of them is a divalent group.

In the present specification, an "adjacent" group may refer to a substituent substituted on an atom directly connected to the atom on which that substituent is substituted, a substituent which is sterically closest to that substituent, or a substituent substituted on the atom on which that substituent is substituted. For example, two substituents substituted at an ortho position on a benzene ring and two substituents substituted at the same carbon on an aliphatic ring may be interpreted as "adjacent" groups to each other.

In the present invention, "when a substituent is not indicated in the chemical formula or compound structure" means that a hydrogen atom is bonded to a carbon atom. However, since deuterium (²H) is an isotope of hydrogen, some hydrogen atoms may be deuterium.

In one embodiment of the present invention, "when a substituent is not indicated in the chemical formula or compound structure" may mean that hydrogen or deuterium is present at all positions that may be substituted with a substituent. That is, deuterium is an isotope of hydrogen, and thus, some hydrogen atoms may be isotope deuterium, and in this case, the content of deuterium may be 0% to 100%.

In one embodiment of the present invention, in the case of "when a substituent is not indicated in the chemical formula or compound structure," hydrogen and deuterium may be used interchangeably in compounds unless deuterium is explicitly excluded, such as "the content of deuterium is 0%," "the content of hydrogen is 100%," and "all substituents are hydrogen".

In one embodiment of the present invention, deuterium is one of the isotopes of hydrogen and is an element having a deuteron consisting of one proton and one neutron as a nucleus, and may be expressed as hydrogen-2, and its element symbol may also be written as D or ²H.

In one embodiment of the present invention, an isotope refers to an atom having the same atomic number (Z) but a different mass number (A), and may also be interpreted as an element having the same number of protons but a different number of neutrons.

In one embodiment of the present invention, the meaning of the T% content of a specific substituent may be defined as the following equation: T2/T1×100 = T%, wherein T1 is defined as the total number of substituents that the basic compound can have and T2 is defined as the number of specific substituents substituted among them.

That is, in one example, the 20% content of deuterium in the phenyl group represented by 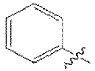 may mean that the total number of substituents that the phenyl group can have is 5 (T1 in the equation) and the number of deuterium among them is 1 (T2 in the equation). That is, the 20% content of deuterium in the phenyl group may be represented by the following structural formulas:

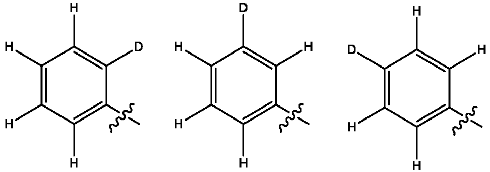

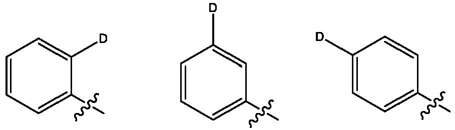

In addition, in one embodiment of the present invention, the case of "a phenyl group having a deuterium content of 0%" may mean a phenyl group that does not contain deuterium atoms, that is, a phenyl group having 5 hydrogen atoms.

In the present invention, the content of deuterium in the heterocyclic compound represented by Formula 1 may be 0 to 100%, more preferably 30 to 100%.

In the present invention, C6 to C60 aromatic hydrocarbon ring refers to a compound including an aromatic ring consisting of C6 to C60 carbons and hydrogens, for example, includes, but is not limited to, benzene, biphenyl, terphenyl, triphenylenyl, naphthalenyl, anthracenyl, phenalenyl, phenanthrenyl, fluorenyl, pyrenyl, chrysenyl, perylenyl, azulenyl, and the like, and includes all of the aromatic hydrocarbon ring compounds known in the art as those satisfying the carbon number described above.

The present invention provides a heterocyclic compound represented by following Formula 1: 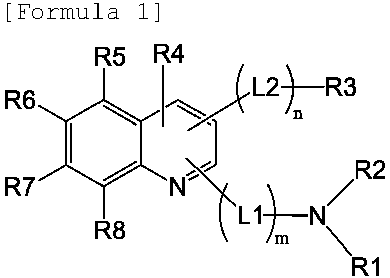 wherein,
L1 and L2 are the same as or different from each other and are each independently a single bond; a substituted or unsubstituted C6 to C60 arylene group; or a substituted or unsubstituted C2 to C60 heteroarylene group,
each of m and n is an integer from 0 to 5, with the proviso that when m is 2 or more, L1 is the same as or different from each other, and when n is 2 or more, L2 is the same as or different from each other,
R1 to R3 are the same as or different from each other and are each independently a substituted or unsubstituted C6 to C60 aryl group or a substituted or unsubstituted C2 to C60 heteroaryl group, and
R4 to R8 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102;-SiR101R102R103; and -NR101R102, or two or more groups adjacent to each other combine with each other to form a substituted or unsubstituted C6 to C60 aromatic hydrocarbon ring or a substituted or unsubstituted C2 to C60 heterocycle, wherein R101, R102, and R103 are the same as or different from each other and are each independently a substituted or unsubstituted C1 to C60 alkyl group; a substituted or unsubstituted C6 to C60 aryl group; or a substituted or unsubstituted C2 to C60 heteroaryl group.

As the aliphatic or aromatic hydrocarbon ring or heterocycle that the adjacent groups may form, the structures exemplified as the above-described cycloalkyl group, cycloheteroalkyl group, aryl group and heteroaryl group may be applied, except for those that are not monovalent groups.

In one embodiment of the present invention, L1 to L2 may be the same as or different from each other and may be each independently a single bond; a substituted or unsubstituted C6 to C30 arylene group; or a substituted or unsubstituted C2 to C30 heteroarylene group.

In another embodiment of the present invention, L1 to L2 may be the same as or different from each other and may be each independently a single bond; a substituted or unsubstituted C6 to C20 arylene group; or a substituted or unsubstituted C2 to C20 heteroarylene group.

In another embodiment of the present invention, L1 to L2 may be the same as or different from each other and may be each independently a single bond; a substituted or unsubstituted C6 to C20 arylene group; or a substituted or unsubstituted carbazolylene group.

In another embodiment of the present invention, L1 to L2 may be the same as or different from each other and may be each independently a single bond; a substituted or unsubstituted phenylene group, naphthylene group or fluorenylene group; or a substituted or unsubstituted carbazolylene group.

In one embodiment of the present invention, each of m and n may be an integer from 1 to 3.

In another embodiment of the present invention, each of m and n may be an integer from 1 to 2.

In one embodiment of the present invention, R1 to R3 may be the same as or different from each other and may be each independently a substituted or unsubstituted C6 to C30 aryl group or a substituted or unsubstituted C2 to C30 heteroaryl group.

In another embodiment of the present invention, R1 to R3 may be the same as or different from each other and may be each independently a substituted or unsubstituted C6 to C20 aryl group or a substituted or unsubstituted C2 to C20 heteroaryl group.

In another embodiment of the present invention, R1 to R3 may be the same as or different from each other and may be each independently a substituted or unsubstituted C6 to C12 aryl group or a substituted or unsubstituted C2 to C12 heteroaryl group.

In another embodiment of the present invention, R1 to R3 may be the same as or different from each other and may be each independently selected from the group consisting of a substituted or unsubstituted phenyl group, naphthyl group, fluorenyl group and phenalenyl group, and a substituted or unsubstituted carbazolyl group, dibenzofuranyl group and dibenzothiophenyl group.

In one embodiment of the present invention, R4 to R8 may be the same as or different from each other and may be each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102;-SiR101R102R103; and -NR101R102, or two or more groups adjacent to each other combine with each other to form a substituted or unsubstituted C6 to C60 aromatic hydrocarbon ring or a substituted or unsubstituted C2 to C60 heterocycle, wherein R101, R102, and R103 may be the same as or different from each other and are each independently a substituted or unsubstituted C1 to C60 alkyl group; a substituted or unsubstituted C6 to C60 aryl group; or a substituted or unsubstituted C2 to C60 heteroaryl group.

In another embodiment of the present invention, R4 to R8 may be the same as or different from each other and may be each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group;-P(=O)R101R102; and -SiR101R102R103, or two or more groups adjacent to each other may combine with each other to form a substituted or unsubstituted C6 to C60 aromatic hydrocarbon ring or a substituted or unsubstituted C2 to C60 heterocycle, wherein R101, R102, and R103 may be the same as or different from each other and are each independently a substituted or unsubstituted C1 to C60 alkyl group; a substituted or unsubstituted C6 to C60 aryl group; or a substituted or unsubstituted C2 to C60 heteroaryl group.

In another embodiment of the present invention, R4 to R8 may be the same as or different from each other and may be each independently hydrogen; deuterium; halogen; cyano group; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C30 aryl group; or a substituted or unsubstituted C2 to C30 heteroaryl group.

In another embodiment of the present invention, R4 to R8 may be the same as or different from each other and may be each independently hydrogen; deuterium; a substituted or unsubstituted C1 to C5 alkyl group; a substituted or unsubstituted C2 to C5 alkenyl group; a substituted or unsubstituted C2 to C5 alkynyl group; a substituted or unsubstituted C6 to C20 aryl group; or a substituted or unsubstituted C2 to C20 heteroaryl group.

In another embodiment of the present invention, R4 to R8 may be the same as or different from each other and may be each independently hydrogen; deuterium; a substituted or unsubstituted C1 to C5 alkyl group; a substituted or unsubstituted C6 to C20 aryl group; or a substituted or unsubstituted C2 to C20 heteroaryl group.

In another embodiment of the present invention, R4 to R8 may be the same as or different from each other and may be each independently hydrogen, deuterium, a substituted or unsubstituted C1 to C5 alkyl group, a substituted or unsubstituted phenyl group, a naphthalenyl group, a pyridinyl group, an anthracenyl group, a carbazolyl group, a dibenzothiophenyl group, a dibenzofuranyl group, or a phenanthrenyl group.

In another embodiment of the present invention, R4 to R8 may be the same as or different from each other and may be each independently hydrogen, deuterium, or a substituted or unsubstituted C1 to C5 alkyl group. In the above, the C1 to C5 alkyl group may be a methyl group, an ethyl group, a linear or branched propyl group, a linear or branched butyl group, or a linear or branched pentyl group.

In another embodiment of the present invention, R4 to R8 may be the same as or different from each other and may be hydrogen or deuterium.

In another embodiment of the present invention, the `substitution' of L1, L2, and R1 to R8 may be each independently made with one or more substituents selected from the group consisting of a C1 to C10 alkyl group; a C2 to C10 alkenyl group; a C2 to C10 alkynyl group; a C3 to C15 cycloalkyl group; a C2 to C20 heterocycloalkyl group; a C6 to C30 aryl group; a C2 to C30 heteroaryl group; a C1 to C10 alkylamine group; a C6 to C30 arylamine group; and a C2 to C30 heteroarylamine group.

In another embodiment of the present invention, the `substitution' of L1, L2, and R1 to R8 may be each independently made with one or more substituents selected from the group consisting of a C1 to C10 alkyl group, a C6 to C30 aryl group, and a C2 to C30 heteroaryl group.

In another embodiment of the present invention, the `substitution' of L1, L2, and R1 to R8 may be each independently made with one or more substituents selected from the group consisting of a C1 to C5 alkyl group, a C6 to C20 aryl group, and a C2 to C20 heteroaryl group.

In another embodiment of the present invention, the `substitution' of L1, L2, and R1 to R8 may be each independently made with one or more substituents selected from the group consisting of a methyl group, an ethyl group, a linear or branched propyl group, a linear or branched butyl group, a linear or branched pentyl group, a phenyl group, a naphthalenyl group, a pyridinyl group, an anthracenyl group, a carbazolyl group, a dibenzothiophenyl group, a dibenzofuranyl group, and a phenanthrenyl group.

In another embodiment of the present invention, the `substitution' of L1, L2, and R1 to R8 may be each independently made with a methyl group, an ethyl group, a linear or branched propyl group, a linear or branched butyl group, or a linear or branched pentyl group.

In one embodiment of the present invention, Formula 1 above may be represented by any one of following Formulas 1-1 to 1-3: 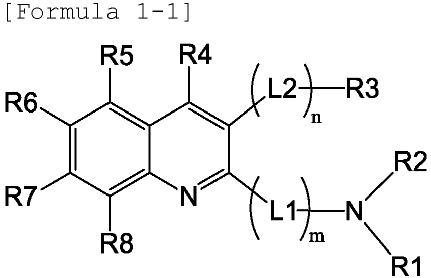 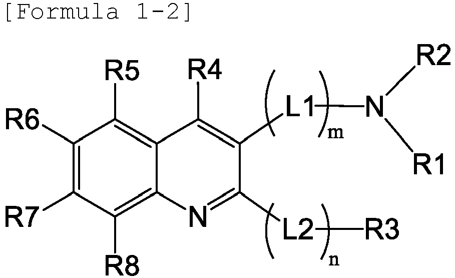 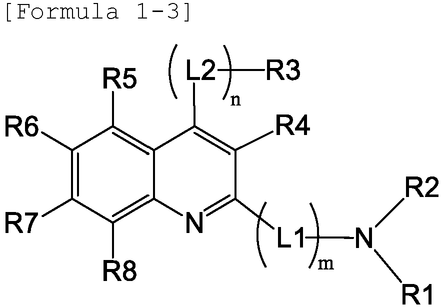 wherein,
L1, L2, R1 to R8, m, and n are the same as defined in Formula 1.

In one embodiment of the present invention, the heterocyclic compound represented by Formula 1 may be one or more selected from the following compounds:

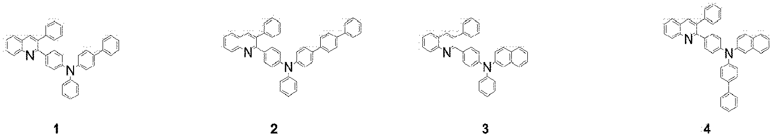

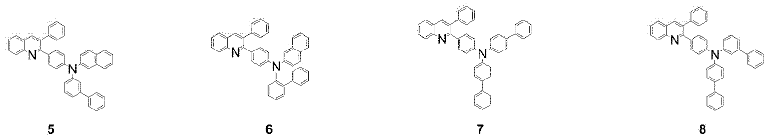

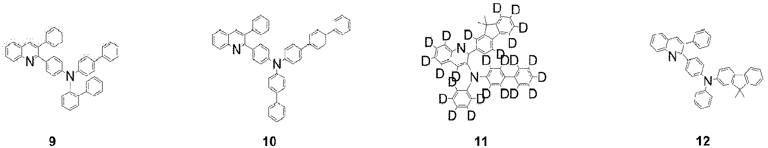

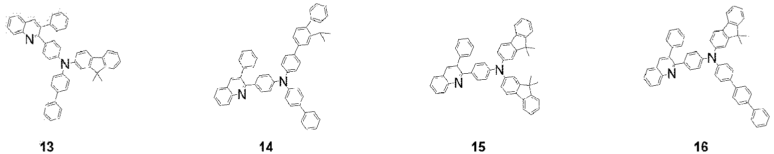

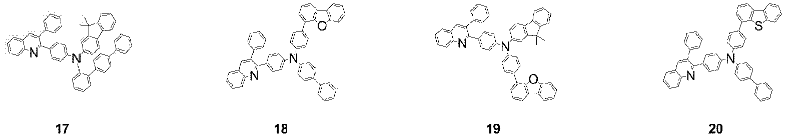

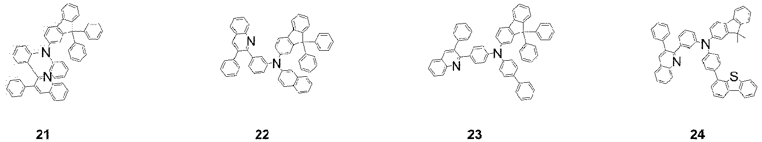

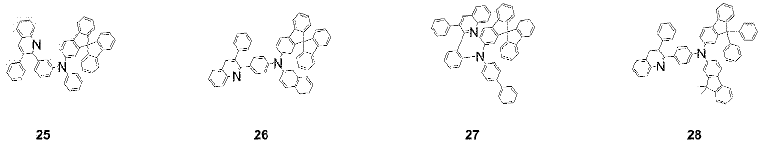

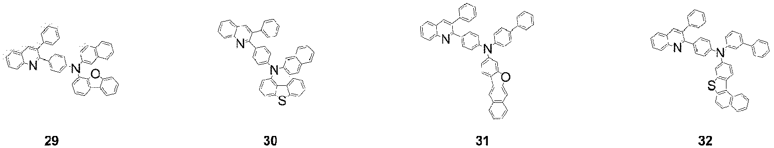

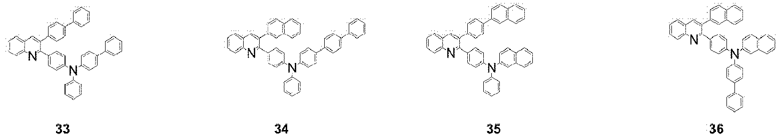

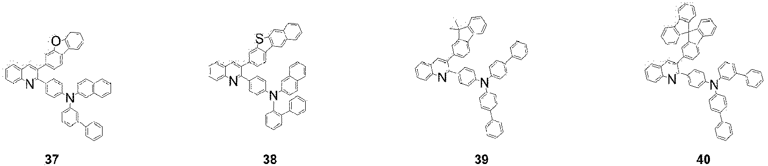

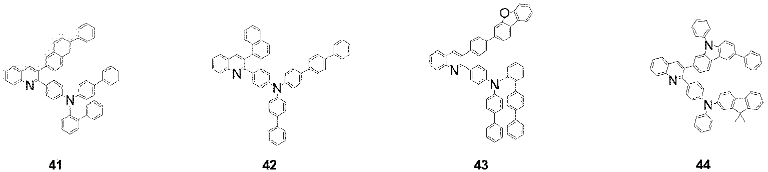

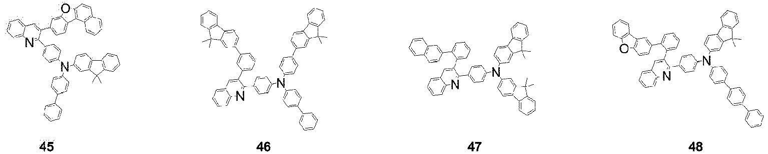

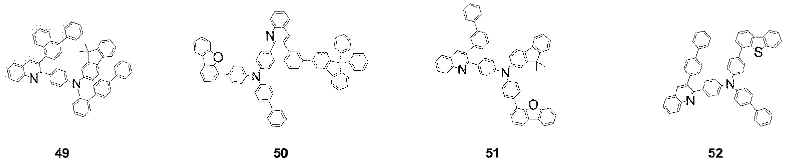

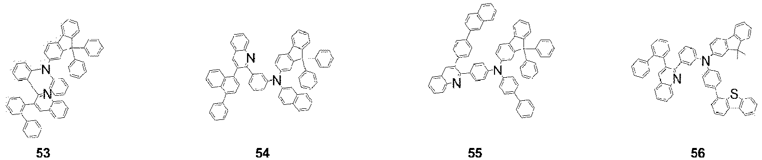

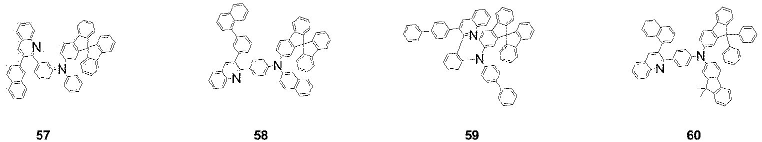

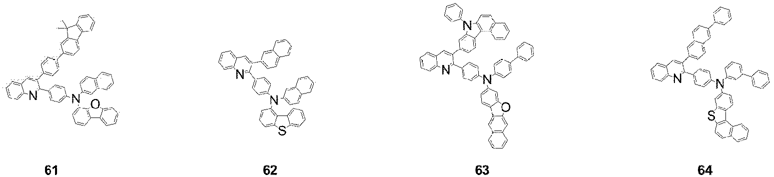

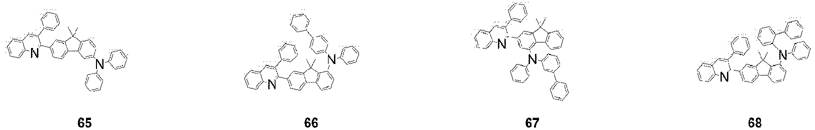

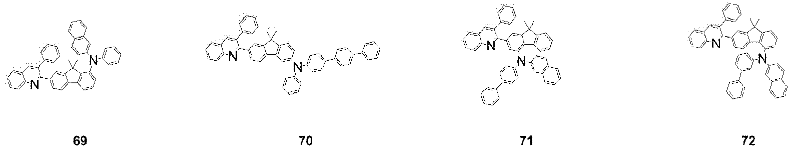

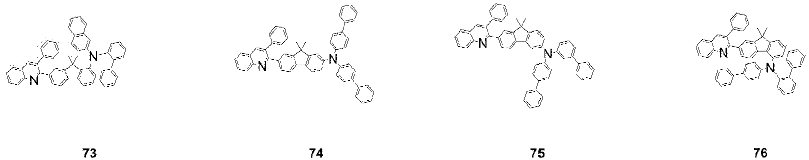

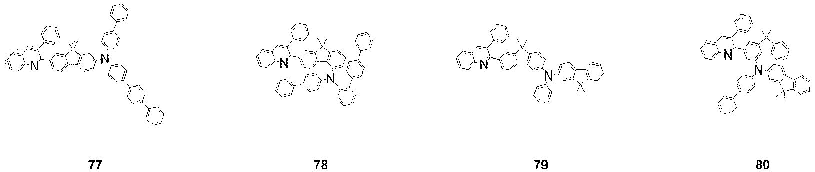

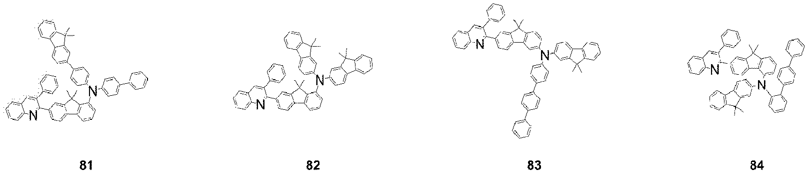

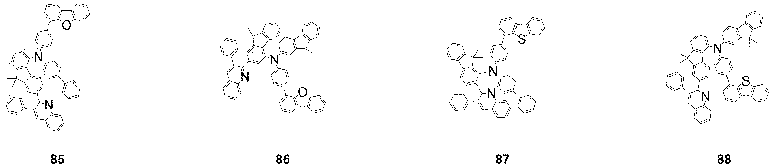

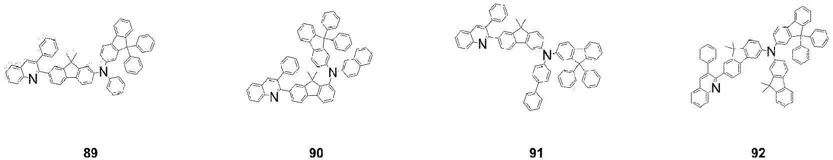

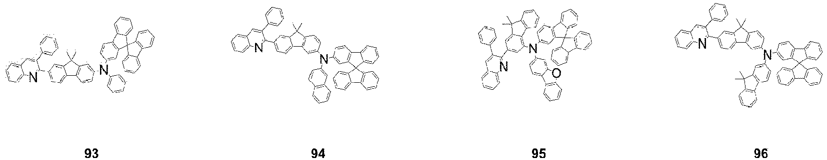

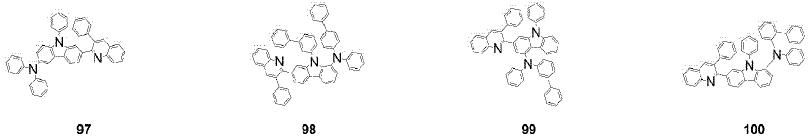

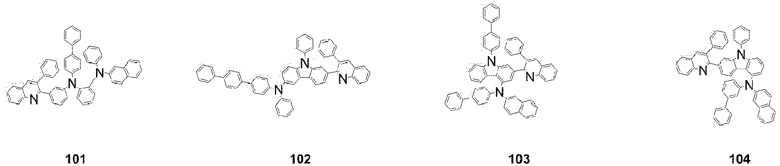

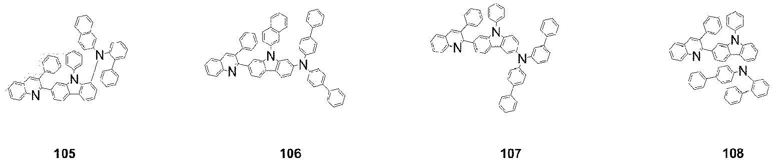

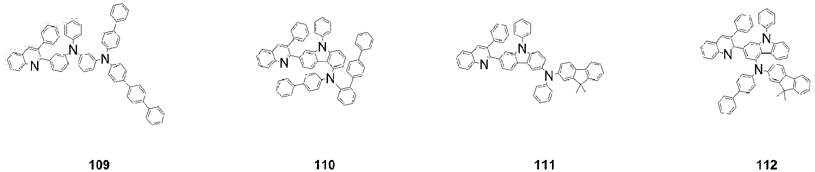

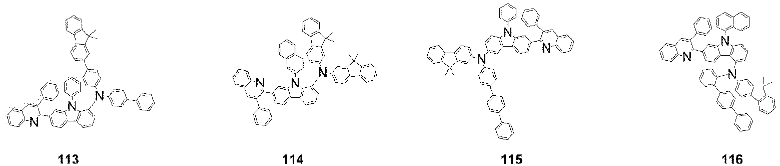

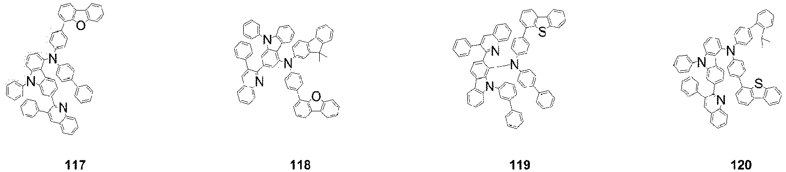

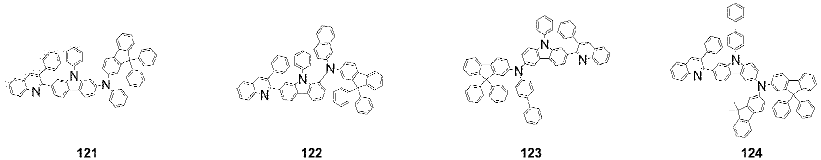

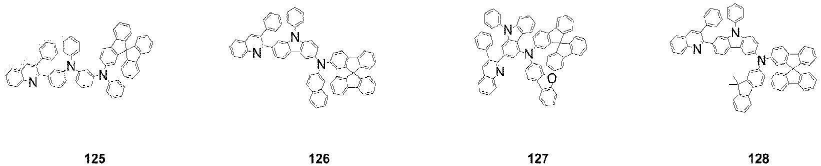

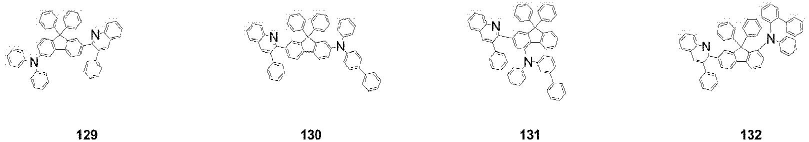

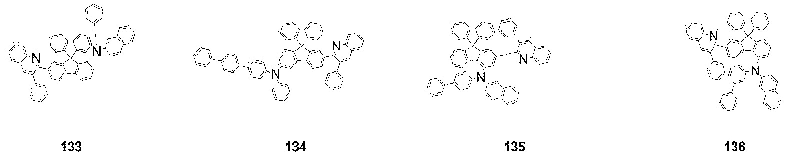

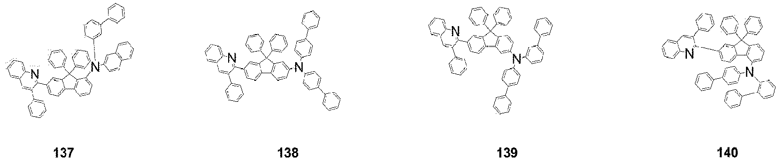

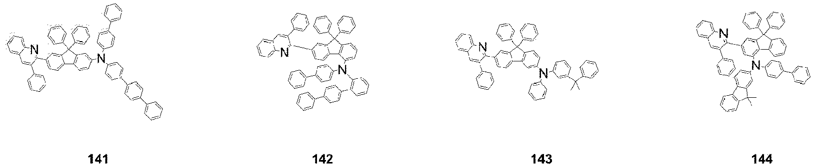

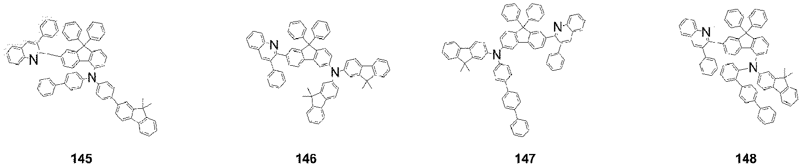

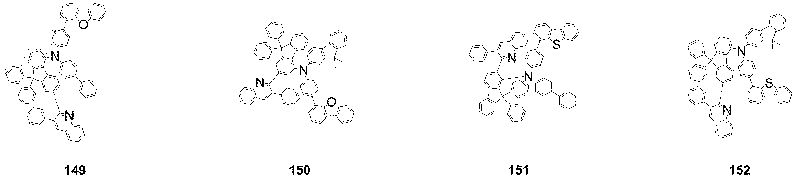

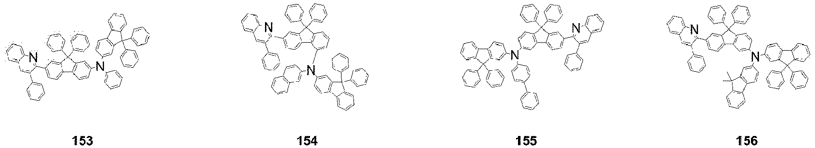

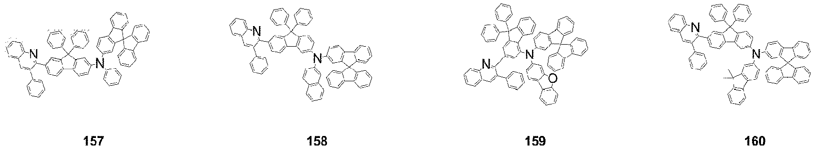

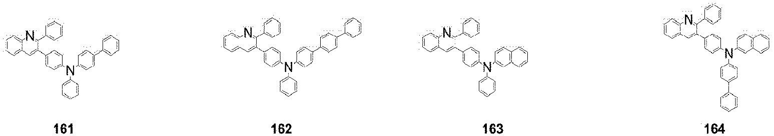

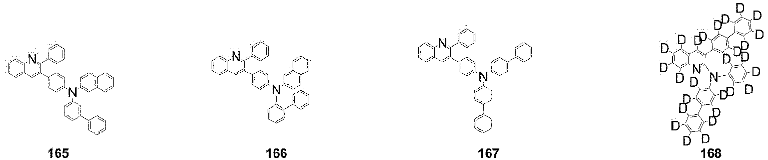

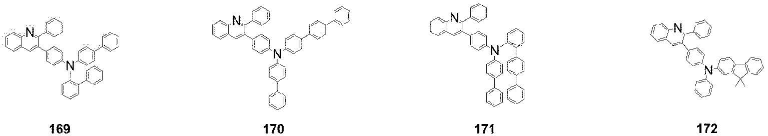

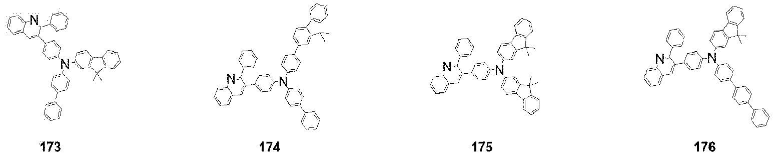

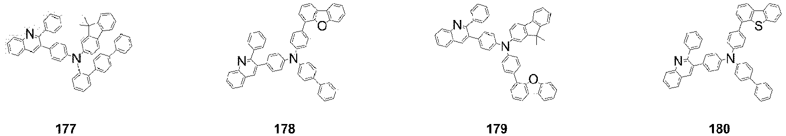

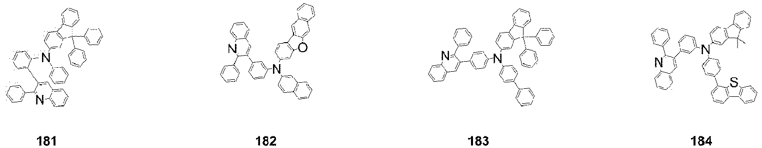

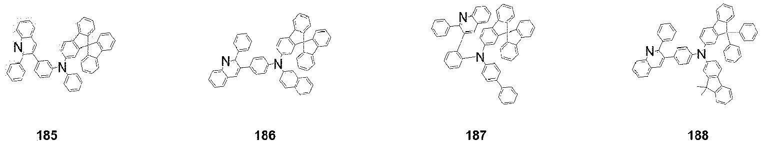

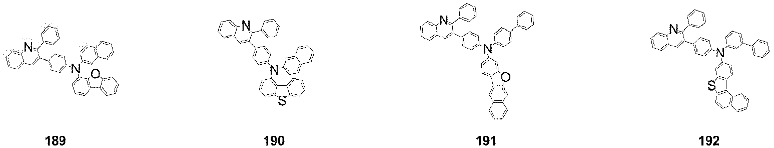

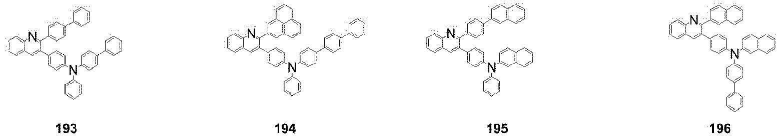

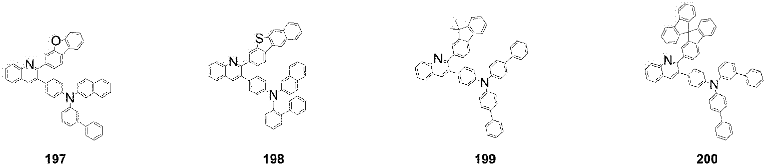

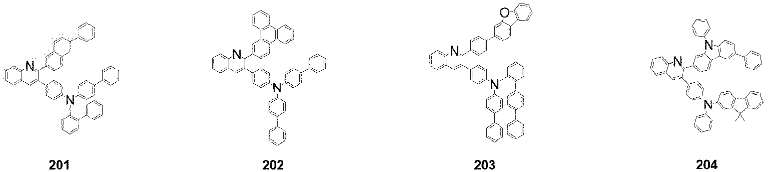

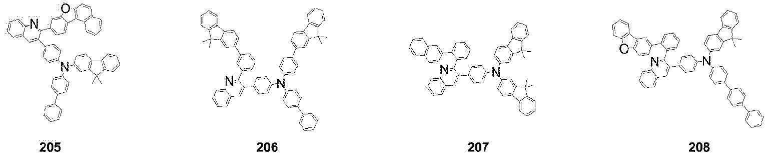

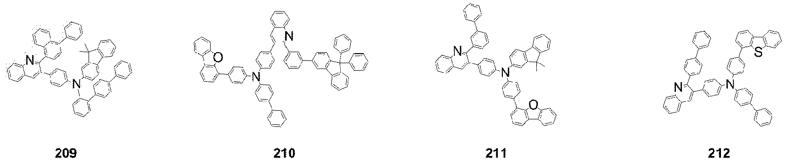

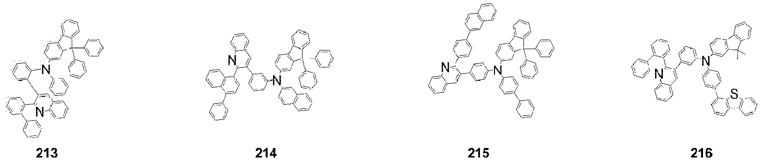

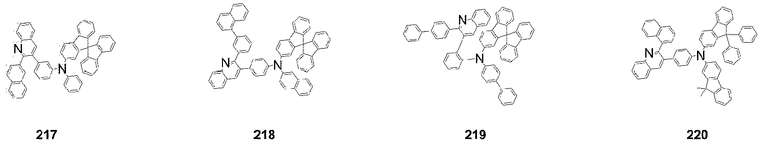

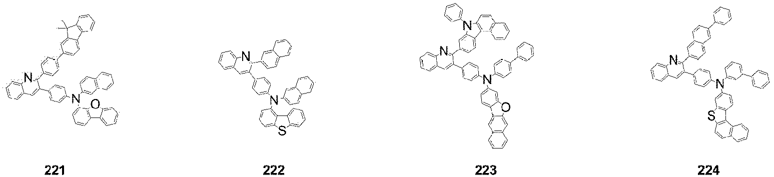

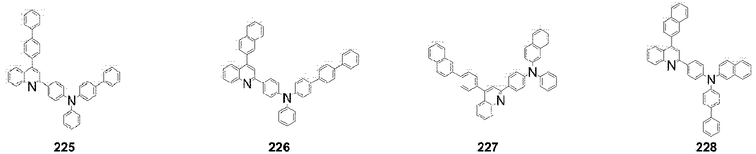

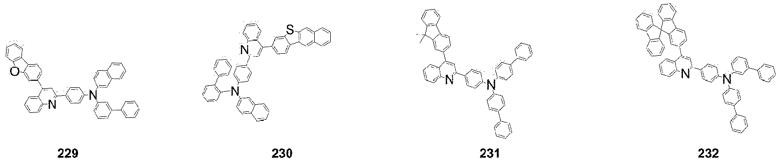

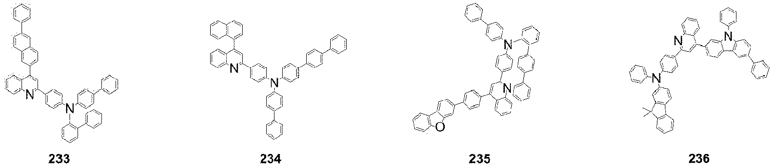

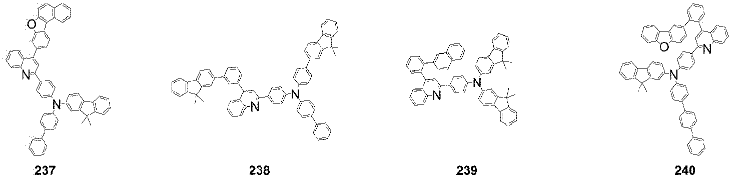

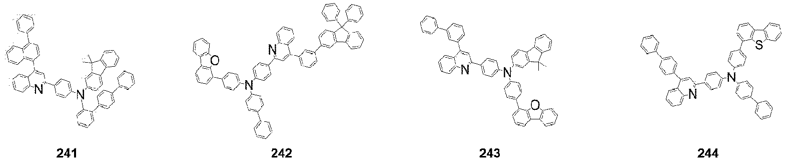

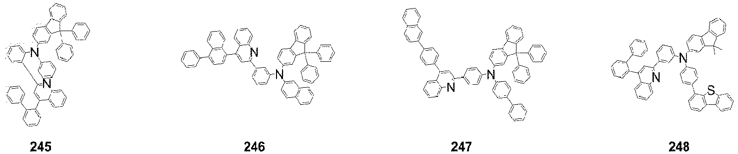

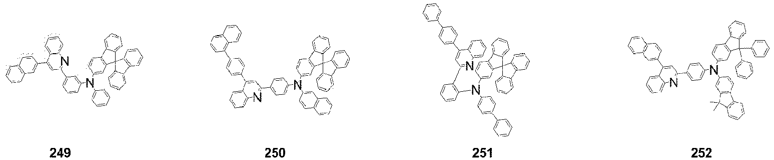

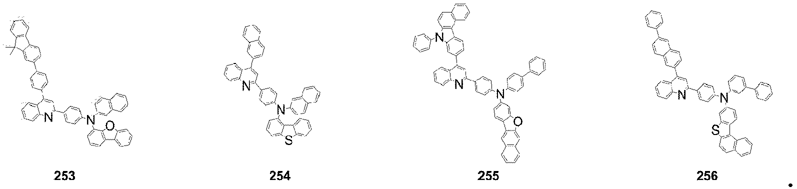

In addition, by introducing various substituents into the structure of Formula 1 above, compounds having intrinsic properties of the introduced substituent may be synthesized. For example, by introducing into the core structure a material for a hole injection layer, a material for a hole transport layer, a material for a light-emitting layer, a material for an electron transport layer, and a material for a charge-generating layer used in manufacturing the organic light-emitting device, it is possible to synthesize materials satisfying the conditions required for each organic layer.

In addition, it is possible to finely control the energy band gap by introducing various substituents into the structure of Formula 1, while it is possible to diversify the use of the materials by improving the properties at the interface between organic materials.

In addition, in one embodiment of the present invention, there is provided an organic light-emitting device comprising a first electrode; a second electrode provided to face the first electrode; and one or more organic layers provided between the first electrode and the second electrode, and wherein at least one of the one or more organic layers comprises the heterocyclic compound represented by Formula 1.

In one embodiment of the present invention, the first electrode may be an anode, and the second electrode may be a cathode.

In another embodiment, the first electrode may be a cathode, and the second electrode may be an anode.

In one embodiment of the present invention, the organic light-emitting device may be a blue organic light-emitting device, and the heterocyclic compound represented by Formula 1 may be used as a material of the blue organic light-emitting device.

In one embodiment of the present invention, the organic light-emitting device may be a green organic light-emitting device, and the heterocyclic compound represented by Formula 1 may be used as a material of the green organic light-emitting device.

In one embodiment of the present invention, the organic light-emitting device may be a red organic light-emitting device, and the heterocyclic compound represented by Formula 1 may be used as a material of the red organic light-emitting device.

In one embodiment of the present invention, the organic light-emitting device may be a blue organic light-emitting device, and the heterocyclic compound represented by Formula 1 may be used as a material for light-emitting layer of the blue organic light-emitting device.

In one embodiment of the present invention, the organic light-emitting device may be a green organic light-emitting device, and the heterocyclic compound represented by Formula 1 may be used as a material for light-emitting layer of the green organic light-emitting device.

In one embodiment of the present invention, the organic light-emitting device may be a red organic light-emitting device, and the heterocyclic compound represented by Formula 1 may be used as a material for light-emitting layer of the red organic light-emitting device.

Specific details of the heterocyclic compound represented by Formula 1 are the same as described above.

The organic light-emitting device of the present invention may be manufactured by conventional methods and materials for manufacturing an organic light-emitting device, except that one or more organic layers are formed using the aforementioned heterocyclic compound.

The heterocyclic compound may be formed into an organic layer by a solution coating method as well as a vacuum deposition method when manufacturing an organic light-emitting device. In this case, the solution coating method refers to, but is not limited to, spin coating, dip coating, inkjet printing, screen printing, spraying, roll coating, and the like.

The organic layer of the organic light-emitting device of the present invention may have a single-layer structure, and may also have a multi-layer structure in which two or more organic layers are stacked. For example, the organic light-emitting device of the present invention may have a structure comprising a hole injection layer, a hole transport layer, a light-emitting layer, an electron transport layer, an electron injection layer, and the like, as an organic layer. However, the structure of the organic light-emitting device is not limited thereto, and may include a smaller number of organic layers.

In the organic light-emitting device according to one embodiment of the present invention, there is provided the organic light-emitting device wherein the organic layer including the heterocyclic compound represented by Formula 1 further comprises the heterocyclic compound represented by Formula 2. 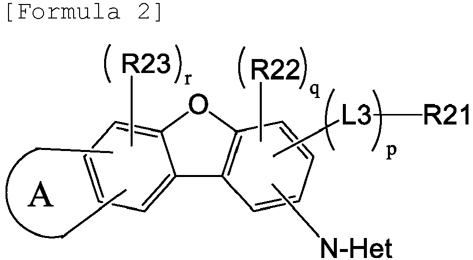 wherein,
N-Het is a substituted or unsubstituted, C2 to C60 monocyclic or polycyclic heterocyclic group containing one or more N,
L3 is a single bond; a substituted or unsubstituted C6 to C60 arylene group; or a substituted or unsubstituted C2 to C60 heteroarylene group, and p is an integer from 0 to 3, with the proviso that when p is 2 or more, L3 is the same as or different from each other,
A is a substituted or unsubstituted C6 to C60 aryl ring or a substituted or unsubstituted C2 to C60 heteroaryl ring,
R21 to R23 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R201R202;-SiR201R202R203; and -NR201R202, or two or more groups adjacent to each other combine with each other to form a substituted or unsubstituted C6 to C60 aromatic hydrocarbon ring or a substituted or unsubstituted C2 to C60 heterocycle, wherein R201, R202, and R203 are the same as or different from each other and are each independently a substituted or unsubstituted C1 to C60 alkyl group; a substituted or unsubstituted C6 to C60 aryl group; or a substituted or unsubstituted C2 to C60 heteroaryl group, and
each of q and r is an integer from 0 to 2, with the proviso that when q is 2, R22 is the same as or different from each other, and when r is 2, R23 is the same as or different from each other.

In one embodiment of the present invention, N-Het may be a substituted or unsubstituted, C2 to C30 monocyclic or polycyclic heterocyclic group containing one or more N.

In another embodiment of the present invention, N-Het may be a substituted or unsubstituted, C3 to C30 monocyclic or polycyclic heterocyclic group containing one or more and three or less N.

In another embodiment of the present invention, N-Het may be a substituted or unsubstituted, C3 to C10 monocyclic or polycyclic heterocyclic group containing one or more and three or less N.

In another embodiment of the present invention, N-Het may be a C2 to C30 monocyclic or polycyclic heterocyclic group unsubstituted or substituted with one or more substituents selected from the group consisting of a C6 to C60 aryl group and a C2 to C60 heteroaryl group and containing one or more N.

In another embodiment of the present invention, N-Het may be a triazinyl group; a pyrimidinyl group; a pyridinyl group; a quinolinyl group; a quinazolinyl group; a phenanthrolinyl group; a imidazolyl group; a benzothiazolyl group; or benzo[4,5]thieno[2,3-d]pyrimidinyl group, which is unsubstituted or substituted with one or more substituents selected from the group consisting of a C6 to C60 aryl group and a C2 to C60 heteroaryl group.

In another embodiment of the present invention, N-Het may be a triazinyl group; a pyrimidinyl group; a pyridinyl group; a quinolinyl group; a quinazolinyl group; a phenanthrolinyl group; a imidazolyl group; a benzothiazolyl group; or benzo[4,5]thieno[2,3-d]pyrimidinyl group, which is unsubstituted or substituted with one or more substituents selected from the group consisting of a phenyl group, a biphenyl group, a naphthyl group, a triphenylenyl group, a dibenzofuranyl group, a dibenzothiophenyl group, a pyridinyl group, a dimethylfluorenyl group, a diphenylfluorenyl group, and a spirobifluorenyl group.

In another embodiment of the present invention, N-Het may be a triazinyl group; a pyrimidinyl group; a quinazolinyl group; or benzo[4,5]thieno[2,3-d]pyrimidinyl group, which is unsubstituted or substituted with one or more substituents selected from the group consisting of a phenyl group, a biphenyl group, a naphthyl group, a triphenylenyl group, a dibenzofuranyl group, a dibenzothiophenyl group, a pyridinyl group, a dimethylfluorenyl group, a diphenylfluorenyl group, and a spirobifluorenyl group.

In another embodiment of the present invention, N-Het may be substituted again with -CN, a phenyl group, P(=O)RR', or SiRR'R".

In one embodiment of the present invention, L3 may be a direct bond, a substituted or unsubstituted C6 to C40 arylene group, or a substituted or unsubstituted C2 to C40 heteroarylene group.

In another embodiment of the present invention, L3 may be a direct bond, a substituted or unsubstituted C6 to C10 arylene group, or a substituted or unsubstituted C2 to C10 heteroarylene group.

In another embodiment of the present invention, L3 may be a direct bond or a substituted or unsubstituted C6 to C10 arylene group.

In another embodiment of the present invention, L3 may be a direct bond, a phenylene group, or a naphthylene group.

In one embodiment of the present invention, p may be an integer from 0 to 3, with the proviso that when p is 2 or more, L3 may be the same as or different from each other.

In one embodiment of the present invention, A may be a substituted or unsubstituted C6 to C40 aryl ring or a substituted or unsubstituted C2 to C40 heteroaryl ring.

In another embodiment of the present invention, A may be a substituted or unsubstituted C6 to C40 aryl ring.

In another embodiment of the present invention, A may be a substituted or unsubstituted benzene ring or a substituted or unsubstituted naphthyl ring.

In another embodiment of the present invention, A may be a benzene ring.

In one embodiment of the present application, "A has a substituted or unsubstituted C6 to C40 aryl ring" means comprising an unsubstituted C6 to C40 aryl ring or a substituted C6 to C40 aryl ring, and the substituent in the substituted C6 to C40 aryl ring includes a form condensed by combining with an adjacent group.

In one embodiment of the present invention, R21 to R23 may be the same as or different from each other, with the proviso that when R22 and R23 are plural, each of R22 and R23 may be the same as or different from each other and may be each independently selected from the group consisting of hydrogen; deuterium; halogen; a cyano group; a substituted or unsubstituted C1 to C10 alkyl group; a substituted or unsubstituted C2 to C10 alkenyl group; a substituted or unsubstituted C2 to C10 alkynyl group; a substituted or unsubstituted C1 to C10 alkoxy group; a substituted or unsubstituted C3 to C20 cycloalkyl group; a substituted or unsubstituted C2 to C20 heterocycloalkyl group; a substituted or unsubstituted C6 to C30 aryl group; a substituted or unsubstituted C2 to C30 heteroaryl group; -P(=O)R201R202; -SiR201R202R203; and -NR201R202, or two or more groups adjacent to each other may combine with each other to form a substituted or unsubstituted C6 to C30 aromatic hydrocarbon ring or a substituted or unsubstituted C2 to C30 heterocycle, wherein R201, R202, and R203 may be the same as or different from each other and are each independently a substituted or unsubstituted C1 to C10 alkyl group; a substituted or unsubstituted C6 to C30 aryl group; or a substituted or unsubstituted C2 to C30 heteroaryl group.

In another embodiment of the present invention, R21 to R23 may be the same as or different from each other, with the proviso that when R22 and R23 are plural, each of R22 and R23 may be the same as or different from each other and may be each independently hydrogen; deuterium; halogen; cyano group; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C30 aryl group; or a substituted or unsubstituted C2 to C30 heteroaryl group.

In another embodiment of the present invention, R21 to R23 may be the same as or different from each other, with the proviso that when R22 and R23 are plural, each of R22 and R23 may be the same as or different from each other and may be each independently hydrogen; deuterium; a substituted or unsubstituted C1 to C5 alkyl group; a substituted or unsubstituted C2 to C5 alkenyl group; a substituted or unsubstituted C2 to C5 alkynyl group; a substituted or unsubstituted C6 to C20 aryl group; or a substituted or unsubstituted C2 to C20 heteroaryl group.

In another embodiment of the present invention, R21 to R23 may be the same as or different from each other, with the proviso that when R22 and R23 are plural, each of R22 and R23 may be the same as or different from each other and may be each independently hydrogen; deuterium; a substituted or unsubstituted C1 to C5 alkyl group; a substituted or unsubstituted C6 to C20 aryl group; or a substituted or unsubstituted C2 to C20 heteroaryl group.

In another embodiment of the present invention, R21 to R23 may be the same as or different from each other, with the proviso that when R22 and R23 are plural, each of R22 and R23 may be the same as or different from each other and may be each independently hydrogen, deuterium, a substituted or unsubstituted C1 to C5 alkyl group, or a substituted or unsubstituted phenyl group, naphthalenyl group, pyridinyl group, anthracenyl group, carbazolyl group, dibenzothiophenyl group, dibenzofuranyl group, or phenanthrenyl group.

In another embodiment of the present invention, R21 to R23 may be the same as or different from each other, with the proviso that when R22 and R23 are plural, each of R22 and R23 may be the same as or different from each other and may be each independently hydrogen, deuterium, or a substituted or unsubstituted C1 to C5 alkyl group. In the above, the C1 to C5 alkyl group may be a methyl group, an ethyl group, a linear or branched propyl group, a linear or branched butyl group, or a linear or branched pentyl group.

In another embodiment of the present invention, R21 to R23 may be the same as or different from each other, with the proviso that when R22 and R23 are plural, each of R22 and R23 may be the same as or different from each other and may be each independently hydrogen or deuterium.

In another embodiment of the present invention, the `substitution' of N-Het, L3, A, and R21 to R23 may be each independently made with one or more substituents selected from the group consisting of a C1 to C10 alkyl group; a C2 to C10 alkenyl group; a C2 to C10 alkynyl group; a C3 to C15 cycloalkyl group; a C2 to C20 heterocycloalkyl group; a C6 to C30 aryl group; a C2 to C30 heteroaryl group; a C1 to C10 alkylamine group; a C6 to C30 arylamine group; and a C2 to C30 heteroarylamine group.

In another embodiment of the present invention, the `substitution' of N-Het, L3, A, and R21 to R23 may be each independently made with one or more substituents selected from the group consisting of a C1 to C10 alkyl group; a C6 to C30 aryl group; and a C2 to C30 heteroaryl group.

In another embodiment of the present invention, the `substitution' of N-Het, L3, A, and R21 to R23 may be each independently made with one or more substituents selected from the group consisting of a C1 to C5 alkyl group; a C6 to C20 aryl group; and a C2 to C20 heteroaryl group.

In another embodiment of the present invention, the `substitution' of N-Het, L3, A, and R21 to R23 may be each independently made with one or more substituents selected from the group consisting of a methyl group, an ethyl group, a linear or branched propyl group, a linear or branched butyl group, a linear or branched pentyl group, a phenyl group, a naphthalenyl group, a pyridinyl group, an anthracenyl group, a carbazolyl group, a dibenzothiophenyl group, a dibenzofuranyl group, and a phenanthrenyl group.

In another embodiment of the present invention, the `substitution' of N-Het, L3, A, and R21 to R23 may be each independently made with a methyl group, an ethyl group, a linear or branched propyl group, a linear or branched butyl group, or a linear or branched pentyl group.

In one embodiment of the present invention, Formula 2 above may be the heterocyclic compound represented by any one of following Formulas 2-1 to 2-3: 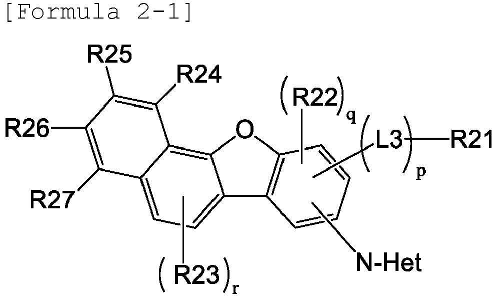 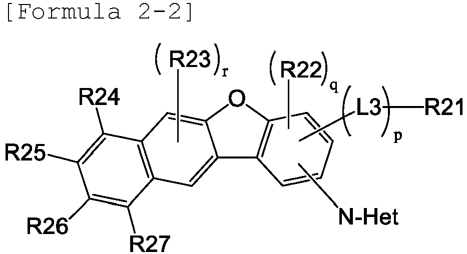 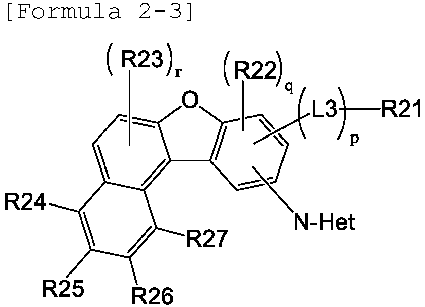 wherein,
R24 to R27 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R201R202;-SiR201R202R203; and -NR201R202, or two or more groups adjacent to each other combine with each other to form a substituted or unsubstituted C6 to C60 aromatic hydrocarbon ring or a substituted or unsubstituted C2 to C60 heterocycle, wherein R201, R202, and R203 are the same as or different from each other and are each independently a substituted or unsubstituted C1 to C60 alkyl group; a substituted or unsubstituted C6 to C60 aryl group; or a substituted or unsubstituted C2 to C60 heteroaryl group, and
N-Het, L3, R21 to R23, p, q, and r are the same as defined in Formula 2.

In one embodiment of the present invention, R24 to R27 may be the same as or different from each other and may be each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R201R202;-SiR201R202R203; and -NR201R202, or two or more groups adjacent to each other may combine with each other to form a substituted or unsubstituted C6 to C60 aromatic hydrocarbon ring or a substituted or unsubstituted C2 to C60 heterocycle, wherein R201, R202, and R203 may the same as or different from each other and are each independently a substituted or unsubstituted C1 to C60 alkyl group; a substituted or unsubstituted C6 to C60 aryl group; or a substituted or unsubstituted C2 to C60 heteroaryl group.

In another embodiment of the present invention, R24 to R27 may be the same as or different from each other and may be each independently hydrogen; deuterium, halogen; cyano group; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C30 aryl group; or a substituted or unsubstituted C2 to C30 heteroaryl group.

In another embodiment of the present invention, R24 to R27 may be the same as or different from each other and may be each independently hydrogen; deuterium; a substituted or unsubstituted C1 to C5 alkyl group; a substituted or unsubstituted C2 to C5 alkenyl group; a substituted or unsubstituted C2 to C5 alkynyl group; a substituted or unsubstituted C6 to C20 aryl group; or a substituted or unsubstituted C2 to C20 heteroaryl group.

In another embodiment of the present invention, R24 to R27 may be the same as or different from each other and may be each independently hydrogen, deuterium, a substituted or unsubstituted C1 to C5 alkyl group, a substituted or unsubstituted C6 to C20 aryl group, or a substituted or unsubstituted C2 to C20 heteroaryl group.

In another embodiment of the present invention, R24 to R27 may be the same as or different from each other and may be each independently hydrogen, deuterium, a substituted or unsubstituted C1 to C5 alkyl group, a substituted or unsubstituted phenyl group, a naphthalenyl group, a pyridinyl group, an anthracenyl group, a carbazolyl group, a dibenzothiophenyl group, a dibenzofuranyl group, or a phenanthrenyl group.

In another embodiment of the present invention, R24 to R27 may be the same as or different from each other and may be each independently hydrogen, deuterium, or a substituted or unsubstituted C1 to C5 alkyl group. In the above, the C1 to C5 alkyl group may be a methyl group, an ethyl group, a linear or branched propyl group, a linear or branched butyl group, or a linear or branched pentyl group.

In another embodiment of the present invention, R24 to R27 may be the same as or different from each other and may be hydrogen or deuterium.

In another embodiment of the present invention, the `substitution' of R24 to R27 may be each independently made with one or more substituents selected from the group consisting of a C1 to C10 alkyl group; a C2 to C10 alkenyl group; a C2 to C10 alkynyl group; a C3 to C15 cycloalkyl group; a C2 to C20 heterocycloalkyl group; a C6 to C30 aryl group; a C2 to C30 heteroaryl group; a C1 to C10 alkylamine group; a C6 to C30 arylamine group; and a C2 to C30 heteroarylamine group.

In another embodiment of the present invention, the `substitution' of R24 to R27 may be each independently made with one or more substituents selected from the group consisting of a C1 to C10 alkyl group; a C6 to C30 aryl group; and a C2 to C30 heteroaryl group.

In another embodiment of the present invention, the `substitution' of R24 to R27 may be each independently made with one or more substituents selected from the group consisting of a C1 to C5 alkyl group; a C6 to C20 aryl group; and a C2 to C20 heteroaryl group.

In another embodiment of the present invention, the `substitution' of R24 to R27 may be each independently made with one or more substituents selected from the group consisting of a methyl group, an ethyl group, a linear or branched propyl group, a linear or branched butyl group, a linear or branched pentyl group, a phenyl group, a naphthalenyl group, a pyridinyl group, an anthracenyl group, a carbazolyl group, a dibenzothiophenyl group, a dibenzofuranyl group, and a phenanthrenyl group.

In another embodiment of the present invention, the `substitution' of R24 to R27 may be each independently made with a methyl group, an ethyl group, a linear or branched propyl group, a linear or branched butyl group, or a linear or branched pentyl group.

In one embodiment of the present invention, N-Het may be the heterocyclic compound represented by any one of following Formulas 3-1 to 3-4: 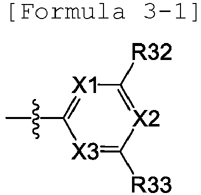 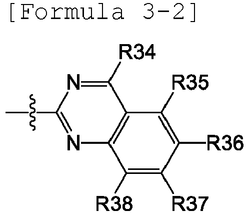 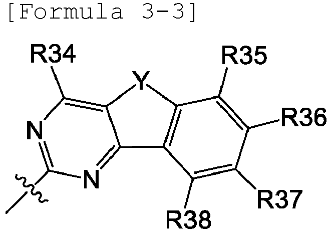 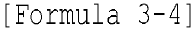 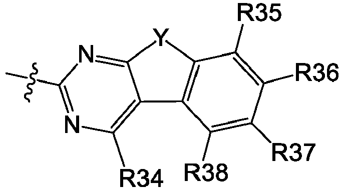 wherein,
X1 to X3 are the same as or different from each other and are each independently N or CR31, and at least two of X1 to X3 are N,
Y is O or S,
R32 to R34 are the same as or different from each other and are each independently a substituted or unsubstituted C6 to C60 aryl group or a substituted or unsubstituted C2 to C60 heteroaryl group, and
R31 and R35 to R38 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R301R302;-SiR301R302R303; and -NR301R302, or two or more groups adjacent to each other combine with each other to form a substituted or unsubstituted C6 to C60 aromatic hydrocarbon ring or a substituted or unsubstituted C2 to C60 heterocycle, wherein R301, R302, and R303 are the same as or different from each other and are each independently a substituted or unsubstituted C1 to C60 alkyl group; a substituted or unsubstituted C6 to C60 aryl group; or a substituted or unsubstituted C2 to C60 heteroaryl group.

In one embodiment of the present invention, X1 to X3 may be the same as or different from each other and may be each independently N or CR31, and two or all of X1 to X3 may be N.

In one embodiment of the present invention, Y may be O or S.

In another embodiment of the present invention, Y may be O.

In another embodiment of the present invention, Y may be S.

In one embodiment of the present invention, R32 to R34 may be the same as or different from each other and may be each independently a substituted or unsubstituted C6 to C40 aryl group or a substituted or unsubstituted C2 to C40 heteroaryl group.

In another embodiment of the present invention, R32 to R34 may be the same as or different from each other and may be each independently a substituted or unsubstituted C6 to C10 aryl group or a substituted or unsubstituted C2 to C10 heteroaryl group.

In another embodiment of the present invention, R32 to R34 may be the same as or different from each other and may be each independently a substituted or unsubstituted phenyl group or naphthyl group, or a substituted or unsubstituted dibenzofuranyl group.

In one embodiment of the present invention, R31 and R35 to R38 may be the same as or different from each other and may be each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R301R302;-SiR301R302R303; and -NR301R302, or two or more groups adjacent to each other may combine with each other to form a substituted or unsubstituted C6 to C60 aromatic hydrocarbon ring or a substituted or unsubstituted C2 to C60 heterocycle, wherein R301, R302, and R303 may be the same as or different from each other and are each independently a substituted or unsubstituted C1 to C60 alkyl group; a substituted or unsubstituted C6 to C60 aryl group; or a substituted or unsubstituted C2 to C60 heteroaryl group.

In another embodiment of the present invention, R31 and R35 to R38 above may be the same as or different from each other and may be each independently hydrogen; deuterium; halogen; cyano group; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C30 aryl group; or a substituted or unsubstituted C2 to C30 heteroaryl group.

In another embodiment of the present invention, R31 and R35 to R38 may be the same as or different from each other and may be each independently hydrogen; deuterium; a substituted or unsubstituted C1 to C5 alkyl group; a substituted or unsubstituted C2 to C5 alkenyl group; a substituted or unsubstituted C2 to C5 alkynyl group; a substituted or unsubstituted C6 to C20 aryl group; or a substituted or unsubstituted C2 to C20 heteroaryl group.

In another embodiment of the present invention, R31 and R35 to R38 may be the same as or different from each other and may be each independently hydrogen; deuterium; a substituted or unsubstituted C1 to C5 alkyl group; a substituted or unsubstituted C6 to C20 aryl group; or a substituted or unsubstituted C2 to C20 heteroaryl group.

In another embodiment of the present invention, R31 and R35 to R38 may be the same as or different from each other and may be each independently hydrogen, deuterium, a substituted or unsubstituted C1 to C5 alkyl group, a substituted or unsubstituted phenyl group, a naphthalenyl group, a pyridinyl group, an anthracenyl group, a carbazolyl group, a dibenzothiophenyl group, a dibenzofuranyl group, or a phenanthrenyl group.

In another embodiment of the present invention, R31 and R35 to R38 may be the same as or different from each other and may be each independently hydrogen, deuterium, or a substituted or unsubstituted C1 to C5 alkyl group. In the above, the C1 to C5 alkyl group may be a methyl group, an ethyl group, a linear or branched propyl group, a linear or branched butyl group, or a linear or branched pentyl group.

In another embodiment of the present invention, R31 and R35 to R38 may be the same as or different from each other and may be hydrogen or deuterium.

In another embodiment of the present invention, the `substitution' of R31 to R38 may be each independently made with one or more substituents selected from the group consisting of a C1 to C10 alkyl group; a C2 to C10 alkenyl group; a C2 to C10 alkynyl group; a C3 to C15 cycloalkyl group; a C2 to C20 heterocycloalkyl group; a C6 to C30 aryl group; a C2 to C30 heteroaryl group; a C1 to C10 alkylamine group; a C6 to C30 arylamine group; and a C2 to C30 heteroarylamine group.

In another embodiment of the present invention, the `substitution' of R31 to R38 may be each independently made with one or more substituents selected from the group consisting of a C1 to C10 alkyl group; a C6 to C30 aryl group; and a C2 to C30 heteroaryl group.

In another embodiment of the present invention, the `substitution' of R31 to R38 may be each independently made with one or more substituents selected from the group consisting of a C1 to C5 alkyl group; a C6 to C20 aryl group; and a C2 to C20 heteroaryl group.

In another embodiment of the present invention, the `substitution' of R31 to R38 may be each independently made with one or more substituents selected from the group consisting of a methyl group, an ethyl group, a linear or branched propyl group, a linear or branched butyl group, a linear or branched pentyl group, a phenyl group, a naphthalenyl group, a pyridinyl group, an anthracenyl group, a carbazolyl group, a dibenzothiophenyl group, a dibenzofuranyl group, and a phenanthrenyl group.

In another embodiment of the present invention, the `substitution' of R31 to R38 may be each independently made with a methyl group, an ethyl group, a linear or branched propyl group, a linear or branched butyl group, or a linear or branched pentyl group.

In one embodiment of the present invention, the heterocyclic compound represented by Formula 2 may be one or more selected from the following compounds:

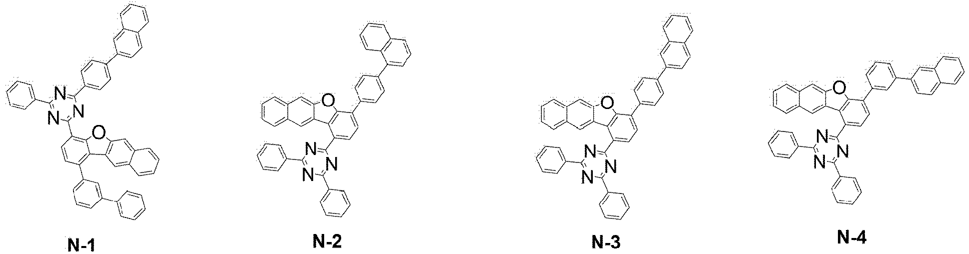

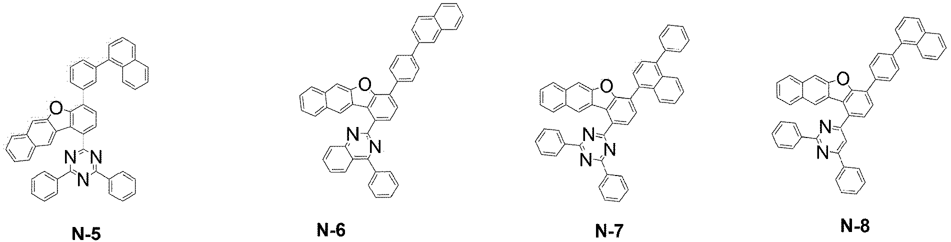

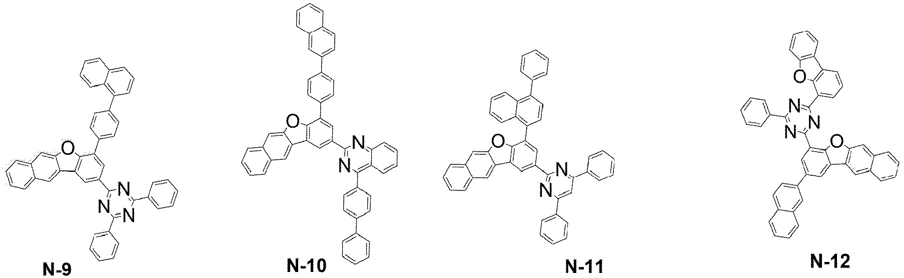

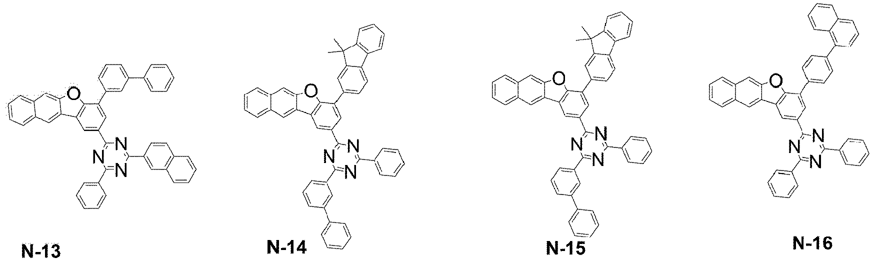

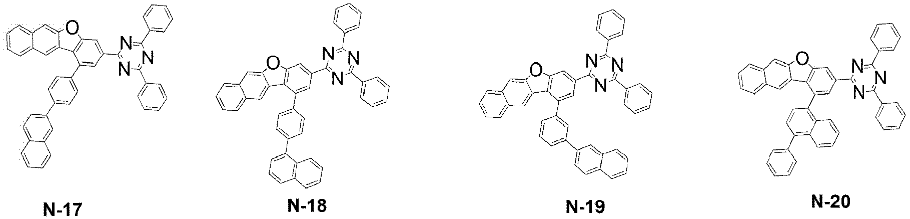

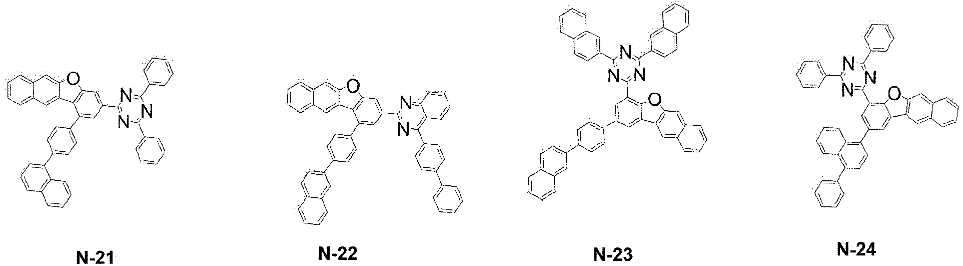

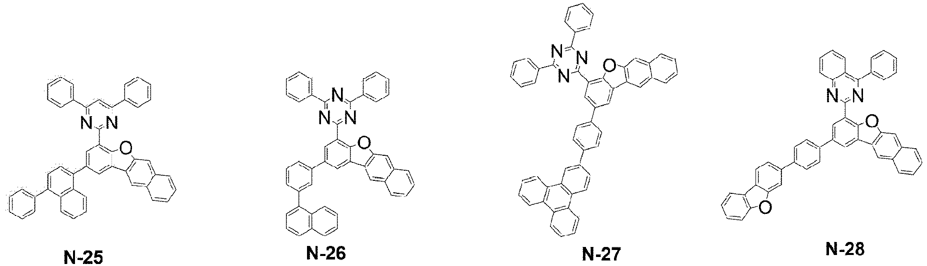

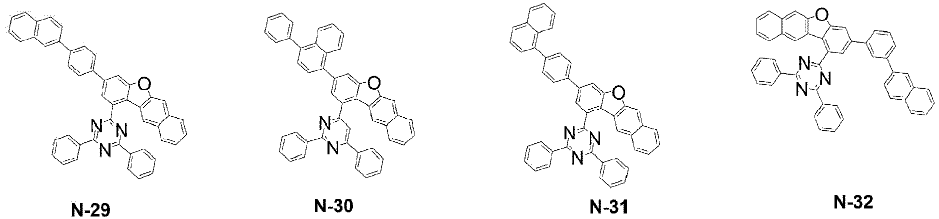

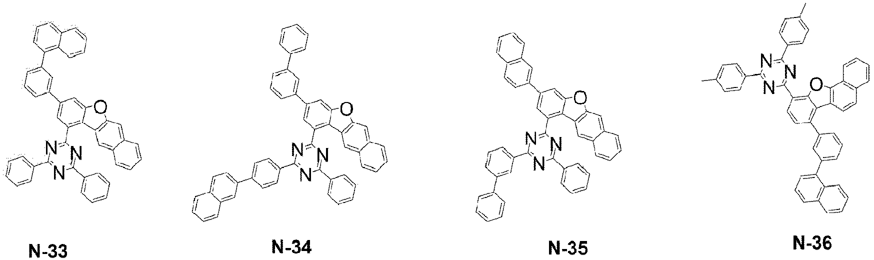

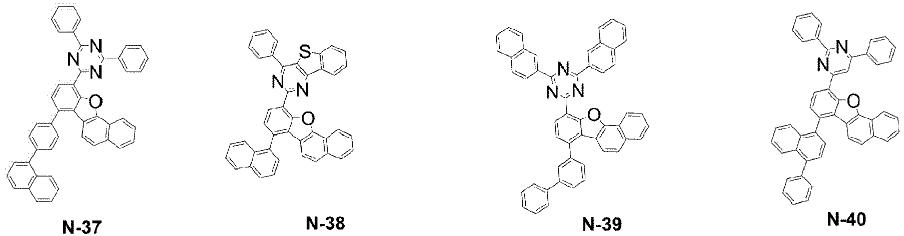

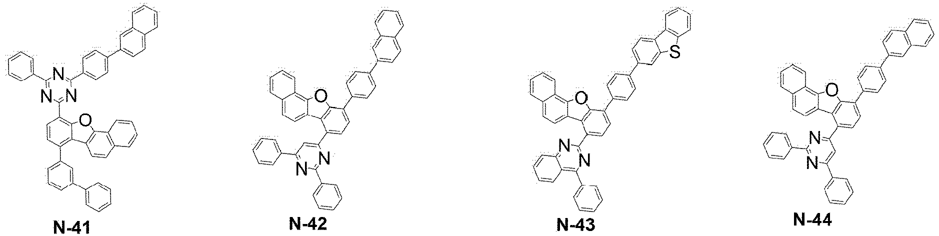

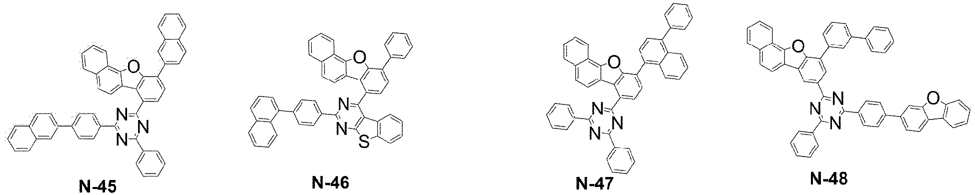

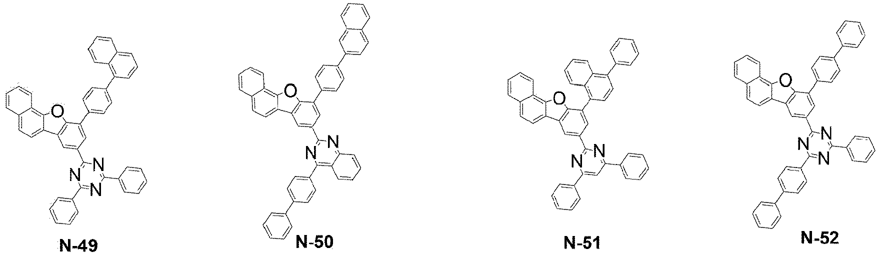

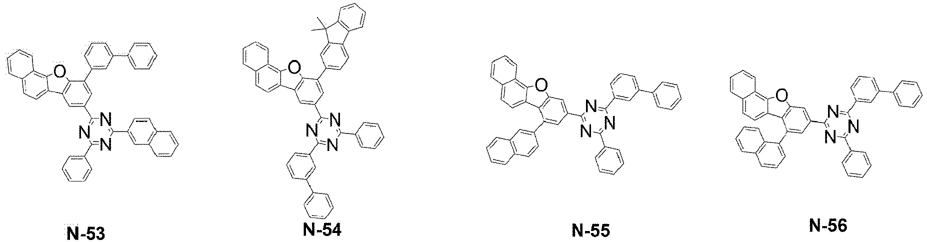

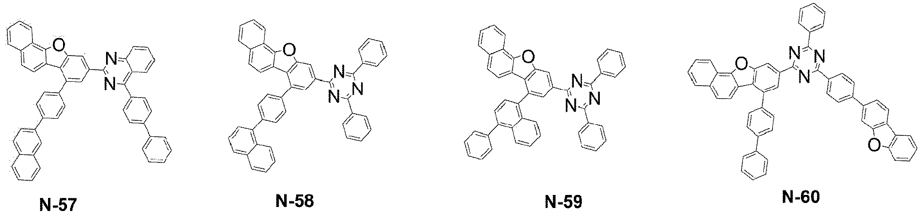

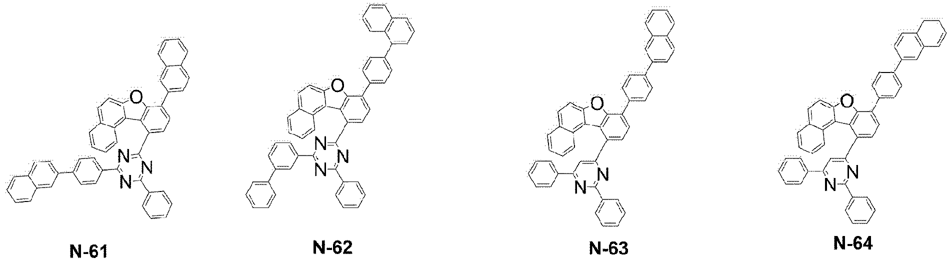

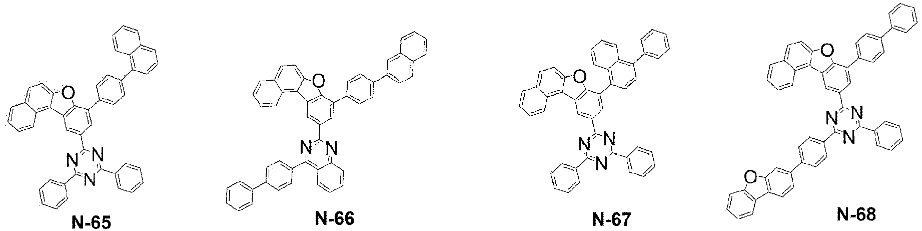

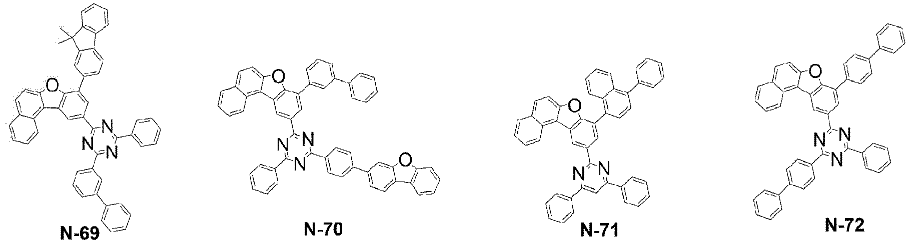

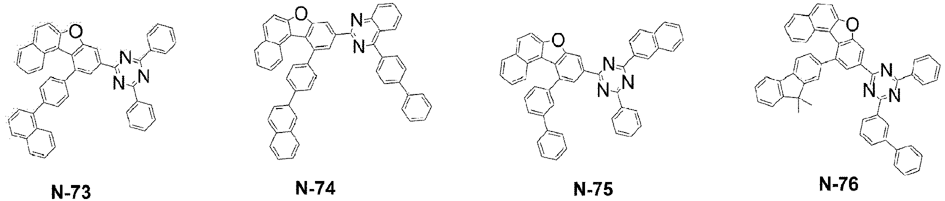

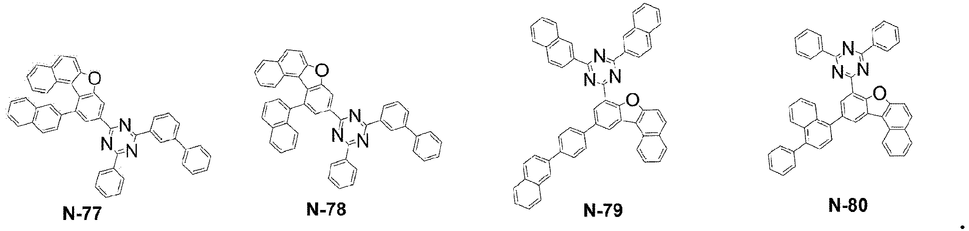

In addition, one embodiment of the present invention provides a composition for an organic layer of the organic light-emitting device comprising the heterocyclic compound represented by Formula 1 and the heterocyclic compound represented by Formula 2.

Specific details of the heterocyclic compound represented by Formula 1 and the heterocyclic compound represented by Formula 2 are the same as described above.

In one embodiment of the present invention, the weight ratio of the heterocyclic compound represented by Formula 1 and the heterocyclic compound represented by Formula 2 in the composition for an organic layer of the organic light-emitting device may be, but is not limited to, 1:10 to 10:1, 1:8 to 8:1, 1:5 to 5:1, or 1:2 to 2:1.

The composition for organic layer of the organic light-emitting device may be used when forming an organic material of the organic light-emitting device, and in particular, may be more preferably used when forming a host of the light-emitting layer.

In one embodiment of the present invention, the organic layer includes the heterocyclic compound represented by Formula 1 and the heterocyclic compound represented by Formula 2, and may be used together with a phosphorescent dopant.

As the phosphorescent dopant material, those known in the art may be used. For example, phosphorescent dopant materials represented by LL'MX', LL'L"M, LMX'X", L₂MX', and L₃M may be used, but the scope of the present invention is not limited by these examples.

M may be iridium, platinum, osmium, or the like.

L is an anionic bidentate ligand coordinated to M by sp² carbon and a heteroatom, and X may function to trap electrons or holes. Non-limiting examples of L include 2-(1-naphthyl)benzoxazole, (2-phenylbenzoxazole), (2-phenylbenzothiazole), (2-phenylbenzothiazole), (7,8-benzoquinoline), (thiophenylpyrizine), phenylpyridine, benzothiophenylpyrizine, 3-methoxy-2-phenylpyridine, tolylpyridine, and the like. Non-limiting examples of X' and X" include acetylacetonate (acac), hexafluoroacetylacetonate, salicylidene, picolinate, 8-hydroxyquinolinate, and the like.

Specific examples of the phosphorescent dopant are shown below, but are not limited to these examples.

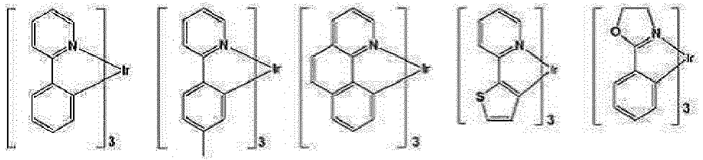

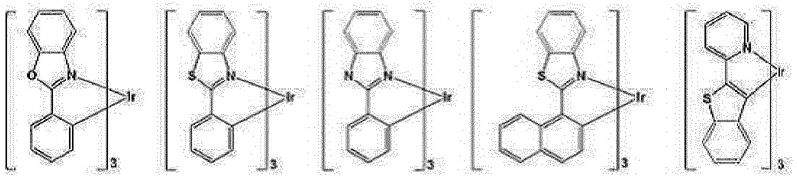

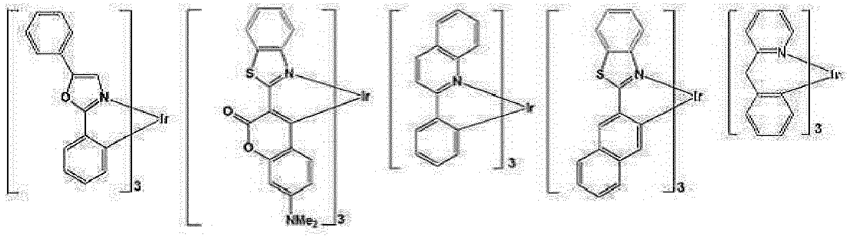

In one embodiment of the present invention, the organic layer includes the heterocyclic compound represented by Formula 1 and the heterocyclic compound represented by Formula 2, and may be used together with an iridium-based dopant.

In one embodiment of the present invention, (piq)₂(Ir) (acac), which is an iridium-based dopant, may be used as a red phosphorescent dopant.

In one embodiment of the present invention, the content of the dopant may be 1% to 15%, preferably 3% to 10% based on the entire light-emitting layer.

In the organic light-emitting device according to one embodiment of the present invention, the organic layer may include an electron injection layer or an electron transport layer, wherein the electron injection layer or the electron transport layer may include the heterocyclic compound.

In the organic light-emitting device according to another embodiment of the present invention, the organic layer may include an electron-blocking layer or a hole-blocking layer, wherein the electron-blocking layer or the hole-blocking layer may include the heterocyclic compound.

In the organic light-emitting device according to another embodiment of the present invention, the organic layer may include an electron transport layer, a light-emitting layer, or a hole-blocking layer, wherein the electron transport layer, the light-emitting layer, or the hole-blocking layer may include the heterocyclic compound.

The organic light-emitting device according to one embodiment of the present invention may further include one or two or more layers selected from the group consisting of a light-emitting layer, a hole injection layer, a hole transport layer, an electron injection layer, an electron transport layer, an electron-blocking layer, and a hole-blocking layer.

Figs. 1 to 3 illustrate the stacking order of the electrodes and the organic layers of the organic light-emitting device according to one embodiment of the present invention. However, it is not intended that the scope of the present application be limited by these drawings, and the structure of the organic light-emitting device known in the art may also be applied to the present application.

According to Fig. 1, there is shown an organic light-emitting device in which an anode 200, an organic layer 300, and a cathode 400 are sequentially stacked on a substrate 100. However, it is not limited only to such a structure, and an organic light-emitting device in which a cathode, an organic layer, and an anode are sequentially stacked on a substrate as shown in Fig. 2 may be embodied.

Fig. 3 illustrates a case where the organic layer is composed of multiple layers. The organic light-emitting device according to Fig. 3 comprises a hole injection layer 301, a hole transport layer 302, a light-emitting layer 303, a hole-blocking layer 304, an electron transport layer 305, and an electron injection layer 306. However, the scope of the present application is not limited by the stacked structures as described above, and the remaining layers except for the light-emitting layer may be omitted, if necessary, and other necessary functional layers may be further added.

In one embodiment of the present invention, there is provided a method of manufacturing an organic light-emitting device, comprising the steps of: preparing a substrate; forming a first electrode on the substrate; forming one or more organic layers on the first electrode; and forming a second electrode on the organic layer, and wherein the step of forming the organic layers comprises a step of forming one or more organic layers using the composition for an organic layer according to one embodiment of the present invention.

In one embodiment of the present invention, the step of forming the organic layers may comprise pre-mixing the heterocyclic compound represented by Formula 1 and the heterocyclic compound represented by Formula 2, and forming the organic layer using a thermal vacuum deposition method.

The pre-mixing refers to mixing the heterocyclic compound represented by Formula 1 and the heterocyclic compound represented by Formula 2 first, putting them in one source, and mixing them, before depositing the materials on the organic layer.

The pre-mixed material may be referred to as a composition for an organic layer according to one embodiment of the present application.

The organic layer including the heterocyclic compound represented by Formula 1 may further comprise another material, if necessary.

The organic layer simultaneously including the heterocyclic compound represented by Formula 1 and Formula 2 may further comprise another material, if necessary.

In the organic light-emitting device according to one embodiment of the present invention, the materials other than the heterocyclic compound represented by Formula 1 or Formula 2 are exemplified below, but these are for illustrative purposes only and are not intended to limit the scope of the present application, and may be replaced with materials known in the art.

As the anode material, materials having a relatively large work function may be used, and transparent conductive oxides, metals, conductive polymers, or the like may be used. Specific examples of the anode material include, but are not limited to, metals such as vanadium, chromium, copper, zinc, and gold, or alloys thereof; metal oxides such as zinc oxide, indium oxide, indium tin oxide (ITO), and indium zinc oxide (IZO); a combination of metals and oxides such as ZnO:Al or SnO₂:Sb; conductive polymers such as poly(3-methylthiophene), poly[3,4-(ethylene-1,2-dioxy)thiophene] (PEDOT), polypyrrole, and polyaniline; and the like.

As the cathode material, materials having a relatively low work function may be used, and metals, metal oxides, conductive polymers, or the like may be used. Specific examples of the cathode material include, but are not limited to, metals such as magnesium, calcium, sodium, potassium, titanium, indium, yttrium, lithium, gadolinium, aluminum, silver, tin, and lead, or alloys thereof; multilayer-structured materials such as LiF/Al or LiO₂/Al; and the like.

As the hole injection layer material, a known material for the hole injection layer may be used, for example, phthalocyanine compounds such as copper phthalocyanine, and the like, disclosed in U.S. Patent No. US 4,356,429, or starburst-type amine derivatives such as tris(4-carbazolyl-9-ylphenyl)amine (TCTA), 4,4',4"-tri[phenyl(m-tolyl)amino]triphenylamine (m-MTDATA), 1,3,5-tris[4-(3-methylphenylphenylamino)phenyl]benzene (m-MTDAPB), soluble conductive polymer polyaniline/dodecylbenzenesulfonic acid or poly(3,4-ethylenedioxythiophene)/poly(4-styrenesulfonate), polyaniline/camphor sulfonic acid or polyaniline/poly(4-styrene-sulfonate), or the like, disclosed in Advanced Material, 6, p.677 (1994) may be used.

As a material for the hole transport layer, a pyrazoline derivative, an arylamine-based derivative, a stilbene derivative, a triphenyldiamine derivative, or the like may be used, and a low-molecular weight or high-molecular weight material may be used.

As a material for the electron transport layer, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone and derivatives thereof, diphenyldicyanoethylene and derivatives thereof, diphenoquinone derivatives, 8-hydroxyquinoline and derivatives thereof, and the like may be used, and high-molecular weight materials as well as low-molecular weight materials may be used.

As a material for the electron injection layer, for example, LiF is typically used in the art, but the present application is not limited thereto.

As a material for the light-emitting layer, a red, green or blue light-emitting material may be used, and a mixture of two or more light-emitting materials may be used, if necessary. In this case, it is possible to use by depositing two or more light-emitting materials as separate sources, or it is possible to use by pre-mixing and depositing them as a single source. In addition, as a material for the light-emitting layer, a fluorescent material may be used, and a phosphorescent material may also be used. As a material for the light-emitting layer, materials that emit light by combining holes and electrons respectively injected from the anode and the cathode may be used alone, and materials in which the host material and the dopant material together participate in light emission may also be used.

When using by mixing hosts of the material for the light-emitting layer, it is possible to use by mixing hosts of the same type, or it is possible to use by mixing different types of hosts. For example, it is possible to use by selecting any two or more types of n-type host materials or p-type host materials as a host material for the light-emitting layer.

The organic light-emitting device according to one embodiment of the present invention may be a top emission type, a bottom emission type, or a dual emission type depending on the material to be used.

The heterocyclic compound according to one embodiment of the present invention may act on the principle similar to that applied to the organic light-emitting device even in an organic electronic device including an organic solar cell, an organic photoreceptor, an organic transistor, and the like.

Hereinafter, preferred examples will be presented to help the understanding of the present invention, but the following examples are provided not to limit the present invention but to facilitate the understanding of the present invention.

### <Preparative Examples>

### <Preparative Example 1> Preparation of Compound 10

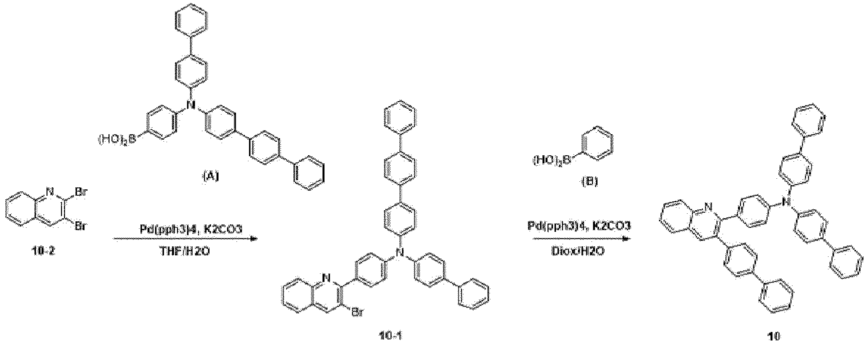

### 1) Preparation of Compound 10-1

400 ml of 1,4-dioxane and 100 ml of H₂O were placed in 20 g (0.070 mol, 1.0 eq) of 2,3-dibromoquinoline (10-2), 36.2 g (0.070 mol, 1.0 eq) of 4-([1,1'-biphenyl]-4-yl([1,1':4',1"-terphenyl]-4-yl)amino)phenyl)boronic acid ((A), 21.3 g (0.154 mol, 2.2 eq) of K₂CO₃, and 4.0 g (0.0035 mol, 0.05 eq) of tetrakis(triphenylphosphine)palladium(0), Pd(PPh₃)₄), and stirred at 100°C for 8 hours. After the reaction was stopped by adding water, extraction was performed using MC and water. Thereafter, water was removed with Mg₂SO₄. It was separated by a silica gel column to obtain 33 g of Compound 10-1 in a yield of 70%.

### 2) Preparation of Compound 10

300 ml of 1,4-dioxane and 60 ml of H₂O were placed in 30 g (0.044 mol, 1.0 eq) of Compound 10-1, 5.4 g (0.044 mol, 1.0 eq) of phenylboronic acid (B), 13.4 g (0.097 mol, 2.2 eq) of K₂CO₃, and 2.5 g (0.0022 mol, 0.05 eq) of tetrakis(triphenylphosphine)palladium(0), (Pd(PPh₃)₄), and stirred at 100°C for 8 hours. After the reaction was stopped by adding water, extraction was performed using MC and water. Thereafter, water was removed with Mg₂SO₄. It was separated by a silica gel column to obtain 21 g of Compound 10 in a yield of 73%.

The following target compound C was prepared in the same manner as in Preparative Example 1 above, except that intermediates A and B of Table 1 below were used instead of (A) and (B), respectively.

**[Table 1]**

| Compound No. | Intermediate A | Intermediate B | Target Compound C | Yield |
|---|---|---|---|---|
| 8 | 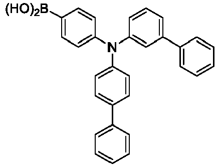 | 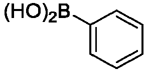 | 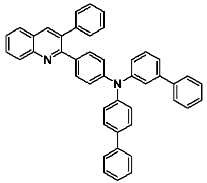 | 57% |
| 16 | 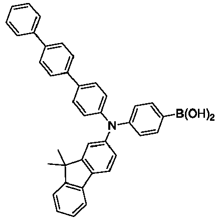 | 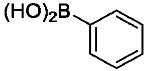 | 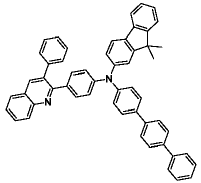 | 52% |
| 59 | 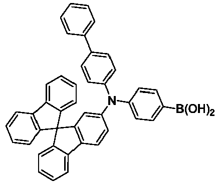 | 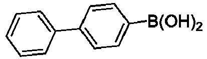 | 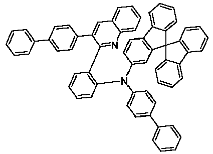 | 50% |
| 63 | 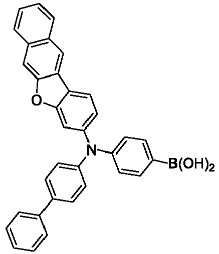 | 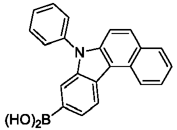 | 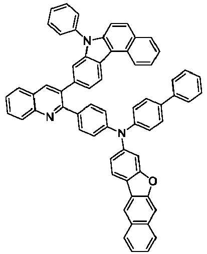 | 52% |
| 70 | 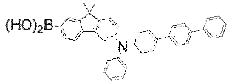 | 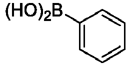 | 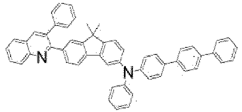 | 55% |
| 87 | 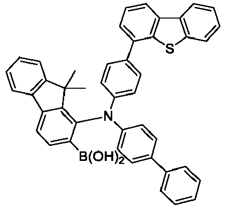 | 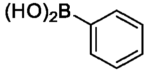 | 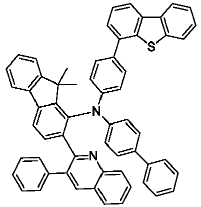 | 50% |
| 117 | 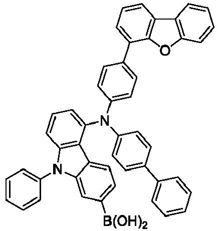 | 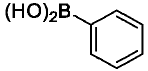 | 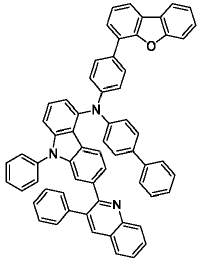 | 53% |
| 170 | 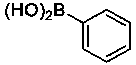 | 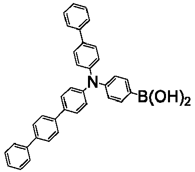 | 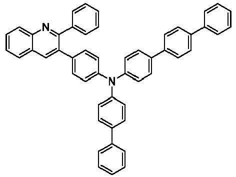 | 61% |
| 200 | 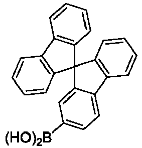 | 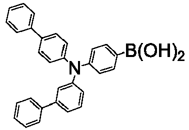 | 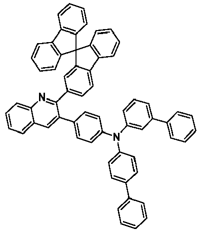 | 64% |
| 206 | 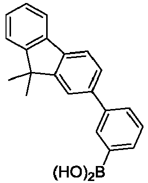 | 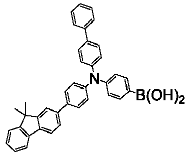 | 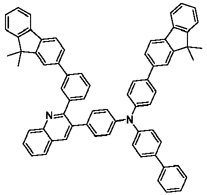 | 57% |
| 215 | 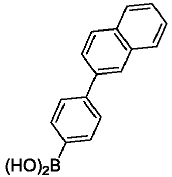 | 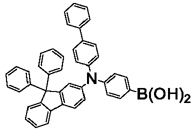 | 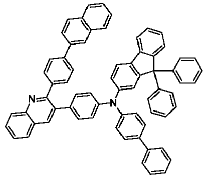 | 60% |

The remaining compounds other than the compounds described in Preparative Example 1 and Table 1 above were prepared in the same manner as in the preparative examples described above, and the synthesis results are shown in Tables 2 and 3 below.

**[Table 2]**

| Compo und | FD-MS | Compo und | FD-MS |
|---|---|---|---|
| 1 | m/z=524.23 (C₃₉H₂₈N₂=524.65) | 3 | m/z=498.21 (C₃₇H₂₆N₂=498.62) |
| 5 | m/z=574.24 (C₄₃H₃₀N₂=574.71) | 7 | m/z=600.26 (C₄₅H₃₂N₂=600.75) |
| 9 | m/z=600.26 (C₄₅H₃₂N₂=600.75) | 11 | m/z=589.41 (C₄₂H₇D₂₅N₂=589.87) |
| 13 | m/z=640.29 (C₄₈H₃₆N₂=640.81) | 15 | m/z=680.32 (C₅₁H₄₀N₂=680.88) |
| 17 | m/z=716.32 (C₅₄H₄₀N₂=716.91) | 19 | m/z=730.30 (C₅₄H₃₈N₂O=730.89) |
| 21 | m/z=688. 29 (C₅₂H₃₆N₂=688.86) | 23 | m/z=764. 32 (C₅₈H₄₀N₂=764.95) |
| 25 | m/z=686.27 (C₅₂H₃₄N₂=686.84) | 27 | m/z=762.30 (C₅₈H₃₈N₂=762.94) |
| 29 | m/z=588.22 (C₄₃H₂₈N₂O=588.70) | 31 | m/z=664.25 (C₄₉H₃₂N₂O=664.79) |
| 33 | m/z=600.26 (C₄₅H₃₂N₂=600.75) | 35 | m/z=624.26 (C₄₇H₃₂N₂=624.77) |
| 37 | m/z=664.25 (C₄₉H₃₂N₂O=664.79) | 39 | m/z=716.32 (C54H₄₀N₂=716.91) |
| 41 | m/z=726.30 (C₅₅H₃₈N₂=726.90) | 43 | m/z=842.33 (C₆₃H₄₂N₂O=843.02) |
| 45 | m/z=780.31 (C₅₈H₄₀N₂O=780.95) | 47 | m/z=806.37 (C₆₁H₄₆N₂=807.03) |
| 49 | m/z=842.37 (C₆₄H₄₆N₂=843.06) | 51 | m/z=806.33 (C₆₀H₄₂N₂O=806.99) |
| 53 | m/z=764.32 (C₅₈H₄₀N₂=764.95) | 55 | m/z=890.37 (C₆₈H₄₆N₂=991.11) |
| 57 | m/z=736.29 (C₅₆H₃₆N₂=736.90) | 59 | m/z=838.33 (C₆₄H₄₂N₂=839.03) |
| 61 | m/z=780.31 (C₅₈H₄₀N₂O=780.95) | 63 | m/z=879.32 (C₆₅H₄₁N₃O=880.04) |
| 65 | m/z=564.26 (C₄₂H₃₂N₂=564.72) | 67 | m/z=640.29 (C₄₈H₃₆N₂=640.81) |
| 69 | m/z=614.27 (C₄₆H₃₄N₂=614.78) | 71 | m/z=690.30 (C₅₂H₃₈N₂=690.87) |
| 73 | m/z=690.30 (C₅₂H₃₈N₂=690.87) | 75 | m/z=716.32 (C₅₄H₄₀N₂=716.91) |
| 77 | m/z=792.35 (C₆₀H₄₄N₂=793.00) | 79 | m/z=680.32 (C₅₁H₄₀N₂=680.88) |
| 81 | m/z=832.38 (C₆₃H₄₈N₂=833.07) | 83 | m/z=832.38 (C₆₃H₄₈N₂=833.07) |
| 85 | m/z=806.33 (C₆₀H₄₂N₂O=806.99) | 87 | m/z=822.31 (C₆₀H₄₂N₂S=823.05) |
| 89 | m/z=804.35 (C₆₁H₄₄N₂=905.02) | 91 | m/z=880.38 (C₆₇H₄₈N₂=881.11) |
| 93 | m/z=802.33 (C₆₁H₄₂N₂=803.00) | 95 | m/z=892.35 (C₆₇H₄₄N₂O=893.08) |
| 97 | m/z=613.25 (C₄₅H₃₁N₃=613.75) | 99 | m/z=689.28 (C₅₁H₃₅N₃=689.84) |
| 101 | m/z=739.30 (C₅₅H₃₇N₃=739.90) | 103 | m/z=815.33 (C₆₁H₄₁N₃=816.00) |
| 105 | m/z=739.30 (C₅₅H₃₇N₃=739.90) | 107 | m/z=765.31 (C₅₇H₃₉N₃=765.94) |
| 109 | m/z=841.35 (C₆₃H₄₃N₃=842.04) | 111 | m/z=729.31 (C₅₄H₃₉N₃=729.91) |
| 113 | m/z=881.38 (C₆₆H₄₇N₃=882.10) | 115 | m/z=881.38 (C₆₆H₄₇N₃=882.10) |
| 117 | m/z=855.32 (C₆₃H₄₁N₃O=856.02) | 119 | m/z=947.33 (C₆₉H₄₅N₃S=948.18) |
| 121 | m/z=853.35 (C₆₄H4₃N₃=854.05) | 123 | m/z=929.38 (C₇₀H₄₇N₃=930.14) |
| 125 | m/z=851.33 (C₆₄H₄₁N₃=852.03) | 127 | m/z=941.34 (C₇₀H₄₃N₃O=942.11) |
| 129 | m/z=688.29 (C52H36N2=688.86) | 131 | m/z=764.32 (C₅₈H₄₀N₂=764.95) |
| 133 | m/z=738.30 (C₅₆H₃₈N₂=738.91) | 135 | m/z=814.33 (C₆₂H₄₂N₂=815.01) |
| 137 | m/z=814.33 (C₆₂H₄₂N₂=815.01) | 139 | m/z=840.35 (C₆₄H₄₄N₂=841.05) |
| 141 | m/z=916.38 (C₇₀H₄₈N₂=917.14) | 143 | m/z=804.35 (C₆₁H₄₄N₂=905.02) |
| 145 | m/z=956.41 (C₇₃H₅₂N₂=957.21) | 147 | m/z=956.41 (C₇₃H₅₂N₂=957.21) |
| 149 | m/z=930.36 (C₇₀H₄₆N₂O=931.13) | 151 | m/z=946.34 (C₇₀H₄₆N₂S=947.19) |
| 153 | m/z=928.38 (C₇₁H₄₈N₂=929.15) | 155 | m/z=1004.41 (C₇₇H₅₂N₂=1005.25) |
| 157 | m/z=926.37 (C₇₁H₄₆N₂=927.14) | 159 | m/z=1016.38 (C₇₇H₄₈N₂O=1017.22) |
| 161 | m/z=524.23 (C₃₉H₂₈N₂=524.65) | 163 | m/z=498.21 (C₃₇H₂₆N₂=498.62) |
| 165 | m/z=574.24 (C₄₃H₃₀N₂=574.71) | 167 | m/z=600.26 (C₄₅H₃₂N₂=600.75) |
| 169 | m/z=600.26 (C₄₅H₃₂N₂=600.75) | 171 | m/z=676.29 (C₅₁H₃₆N₂=676.84) |
| 173 | m/z=640.29 (C₄₈H₃₆N₂=640.81) | 175 | m/z=680.32 (C₅₁H₄₀N₂=680.88) |
| 177 | m/z=716.32 (C₅₄H₄₀N₂=716.91) | 179 | m/z=730.30 (C₅₄H₃₈N₂O=730.89) |
| 181 | m/z=688.29 (C₅₂H₃₆N₂=688.86) | 183 | m/z=764.32 (C₅₈H₄₀N₂=764.95) |
| 185 | m/z=686.27 (C₅₂H₃₄N₂=686.84) | 187 | m/z=762.30 (C₅₈H₃₈N₂=762.94) |
| 189 | m/z=588.22 (C₄₃H₂₈N₂O=588.70) | 191 | m/z=664.25 (C₄₉H₃₂N₂O=664.79) |
| 193 | m/z=600.26 (C₄₅H₃₂N₂=600.75) | 195 | m/z=624.26 (C₄₇H₃₂N₂=624.77) |
| 197 | m/z=664.25 (C₄₉H₃₂N₂O=664.79) | 199 | m/z=716.32 (C54H₄₀N₂=716.91) |
| 201 | m/z=726.30 (C₅₅H₃₈N₂=726.90) | 203 | m/z=842.33 (C₆₃H₄₂N₂₀=843.02) |
| 205 | m/z=780.31 (C₅₈H₄₀N₂O=780.95) | 207 | m/z=806.37 (C₆₁H₄₆N₂=807.03) |
| 209 | m/z=842.37 (C₆₄H₄₆N₂=843.06) | 211 | m/z=806.33 (C₆₀H₄₂N₂O=806.99) |
| 213 | m/z=764.32 (C₅₈H₄₀N₂=764.95) | 215 | m/z=890.37 (C₆₈H₄₆N₂=891.11) |
| 217 | m/z=736.29 (C₅₆H₃₈N₂=736.90) | 219 | m/z=838.33 (C₆₄H₄₂N₂=839.03) |
| 221 | m/z=780. 31 (C₅₈H₄₀N₂O=780.95) | 223 | m/z=879. 32 (C₆₅H₄₁N₃O=880.04) |
| 225 | m/z=600.26 (C₄₅H₃₂N₂=600.75) | 227 | m/z=624.26 (C₄₇H₃₂N₂=624.77) |
| 229 | m/z=664.25 (C₄₉H₃₂N₂O=664.79) | 231 | m/z=716.32 (C54H₄₀N₂=716.91) |
| 233 | m/z=726.30 (C₅₅H₃₈N₂=726.90) | 235 | m/z=842.33 (C₆₃H₄₂N₂O=843.02) |
| 237 | m/z=780.31 (C₅₈H₄₀N₂O=780.95) | 239 | m/z=806.37 (C₆₁H₄₆N₂=807.03) |
| 241 | m/z=842.37 (C₆₄H₄₆N₂=843.06) | 243 | m/z=806.33 (C₆₀H₄₂N₂O=806.99) |
| 245 | m/z=764.32 (C₅₈H₄₀N₂=764.95) | 247 | m/z=890.37 (C₆₈H₄₆N₂=891.11) |
| 249 | m/z=736.29 (C₅₆H₃₈N₂=736.90) | 251 | m/z=838.33 (C₆₄H₄₂N₂=839.03) |
| 253 | m/z=780.31 (C₅₈H₄₀N₂O=780.95) | 255 | m/z=879.32 (C₆₅H₄₁N₃O=880.04) |

**[Table 3]**

| Compound | ¹H NMR (CDCl₃, 200Mz) |
|---|---|
| 2 | δ= 8.06-8.05(3H, m), 7.98(1H, d), 7.94(1H, d), 7.78(1H, t), 7.60-7.41(11H, m), 7.25-7.19(8H, m), 6.81(1H, t), 6.63(5H, m), 6.72-7.69 (4H, m), 6.63 (2H, d) |
| 10 | δ= 8.06-7.94(5H, m), 7.78(1H, t), 7.60~7.41(18H, m), 7.25(4H, s), 7.19(2H, d), 6.72-6.69(6H, m) |
| 18 | δ= 8.06-7.78(9H, m), 7.66-7.32(17H, m), 7.19(2H, d), 6.72-6.69(6H, m) |
| 23 | δ= 8.06-7.94(5H, m), 7.87(1H, d), 7.78(1H, t), 7.62-7.11(27H, m), 6.75-6.69(5H, m), 6.58(1H, d) |
| 34 | δ= 8.06-7.92(8H, m), 7.78-73(2H, m), 7.60-7.41(11H, m), 7.25(4H, s), 6.81(1H, m), 6.72-6.63(6H, m) |
| 42 | δ= 8.55(1H, d), 8.42(1H, d), 8.08-7.94(7H, m), 7.78(1H, t), 7.60-7.41(18H, m), 7.25(4H, s), 6.72-6.69(6H, m) |
| 57 | δ= 8.06-7.87(7H, m), 7.78-7.16(23H, m), 6.81-6.75(2H, m), 6.65-6.58(4H, m) |
| 63 | δ= 8.54(1H, d), 8.49(1H, d), 8.16-7.94(9H, m), 7.78(1H, t), 7.67-7.41(24H, m), 6.72-6.69(4H, m), 6.33(1H, d) |
| 76 | δ= 8.28(1H, d), 8.14(1H, d), 8.06(1H, d), 7.98~7.94(3H, m), 7.78(1H, t), 7.60-7.41(15H, m), 7.19-7.03(6H, m), 6.91(1H, m), 6.69(2H, d), 6.58(1H, d), 1.72(6H, s) |
| 97 | δ= 8.61(1H, d), 8.52(1H, d), 8.13(1H, s), 8.06(1H, d), 7.98-7.94(2H, m), 7.78(1H, t), 7.58-7.38(10H, m), 7.20-7.19(6H, m), 6.81-6.75(4H, m), 6.63(4H, d) |
| 117 | δ= 8.61(1H, d), 8.52(1H, d), 8.13(1H, s), 8.06(1H, d), 7.98-7.78(6H, m), 7.66-7.32(22H, m), 7.19(2H, d), 7.12(1H, t), 6.99(1H, d), 6.69(4H, d) |
| 127 | δ= 8.12(1H, d), 8.06(1H, d), 7.94-7.75(8H, m), 7.66-7.16(30H, m), 6.75(1H, s), 6.58(1H, d), 6.33(1H, d) |
| 142 | δ= 8.28(1H, s), 8.14(1H, d), 8.06(1H, d), 7.98-7.94(3H, m), 7.78(1H, t), 7.60-7.03(35H, m), 6.91-6.87(2H, m), 6.69(2H, d), 6.58(1H, d) |
| 162 | δ= 8.30(2H, d), 8.06(1H, d), 7.98-7.94(2H, m), 7.78(1H, t), 7.60-7.41(13H, m), 7.25(4H, s), 7.20(2H, t), 6.81(1H, t), 6.69(4H, d), 6.63(2H, d) |
| 178 | δ= 8.30(2H, d), 8.06(1H, d), 7.98-7.78(6H, m), 7.66-7.32(19H, m), 6.69(6H, d) |
| 191 | δ= 8.30(2H, d), 8.16(2H, m), 8.06(1H, d), 7.98-7.94(2H, m), 7.78(1H, t), 7.67-7.41(19H, m), 6.69(4H, d), 6.33(1H, d) |
| 246 | δ= 8.55(2H, d), 8.06-7.98(4H, m), 7.88-7.74(8H, m), 7.66-7.26(23H, m), 7.11(4H, d), 6.75(1H, s), 6.65(1H, d), 6.58(1H, d) |

### <Preparative Example 2> Preparation of Compound N-3

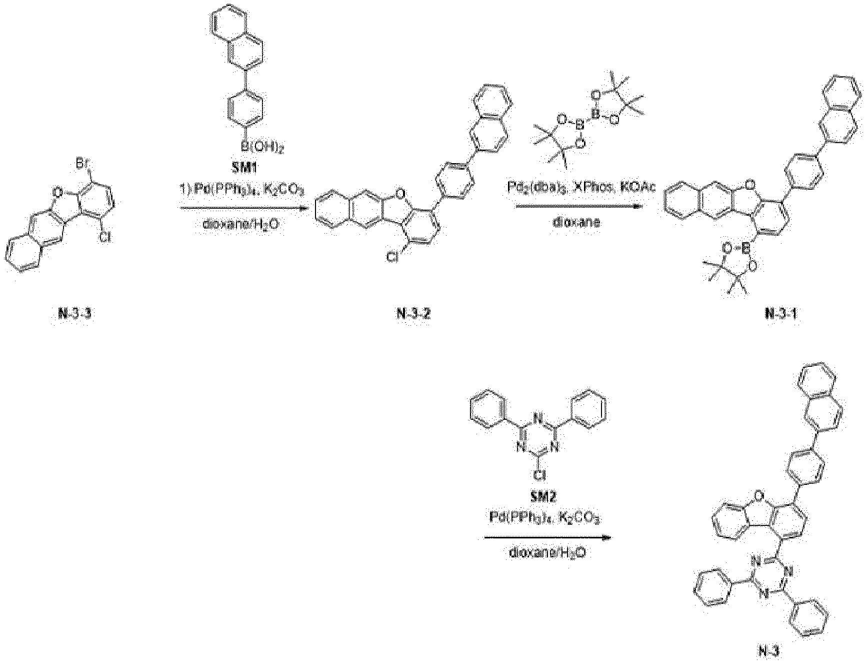

### 1) Preparation of Compound N-3-2

5 g (13.5 mmol) of Compound N-3-3, 4.6 g (16.2 mmol) of Compound SM1, 0.8 g (0.67 mmol) of Pd(PPh₃)₄ (tetrakis(triphenylphosphine)palladium(0)), and 3.7 g (27 mmol) of K₂CO₃ were placed in a 250 mL round-bottom flask, 80 mL of 1,4-dioxane and 20 mL of H₂O were placed therein under a nitrogen atmosphere, and stirred at 120°C for 12 hours. After the reaction was stopped, the reaction temperature was lowered to room temperature, and it was washed with water and extracted with methylchloride (MC). The extracted organic solvent was dried over Mg₂SO₄ and then concentrated. Silica gel column purification and recrystallization were performed to obtain 2.9 g (6.5 mmol, yield 48%) of Compound N-3-2 as a white solid compound.

### 2) Preparation of Compound N-3-1

2.9 g (6.5 mmol) of Compound N-3-2, 2.5 g (9.8 mmol) of 4,4,4',4',5,5,5',5'-octamethyl-2,2'-bi(1,3,2-)dioxaborolane compound, 0.3 g (0.3 mmol) of Pd₂(dba)₃ (tris(dibenzylideneacetone)dipalladium(0)), 1.2 g (2.6 mmol) of XPhos (2-dicyclohexylphosphino-2',4',6'-triisopropylbiphenyl), and 1.3 g (13 mmol) of KOAc were placed in a 100 mL round-bottom flask, and 50 mL of 1,4-dioxane was placed therein under a nitrogen atmosphere, and stirred at 110°C for 3 hours. After the reaction was stopped, the reaction temperature was lowered to room temperature, and it was washed with water and extracted with methylchloride (MC). The extracted organic solvent was dried over Mg₂SO₄ and then concentrated. Silica gel column purification and recrystallization were performed to obtain 3.0 g (5.54 mmol, yield 85%) of Compound N-3-1 as a white solid compound.

### 3) Preparation of Compound N-3

3.0 g (5.54 mmol) of Compound N-3-1, 1.8 g (6.6 mmol) of Compound SM2, 0.3 g (0.27 mmol) of Pd(PPh₃)₄, and 1.5 g (11 mmol) of K₂CO₃ were placed in a 100 mL round-bottom flask, 40 mL of 1,4-dioxane and 10 mL of H₂O were placed therein under a nitrogen atmosphere, and stirred at 120°C for 4 hours. After the reaction was stopped, the reaction temperature was lowered to room temperature, and it was washed with water and extracted with methylchloride. The extracted organic solvent was dried over Mg₂SO₄ and then concentrated. Silica gel column purification and recrystallization were performed to obtain 3.1g (4.7 mmol, yield 85%) of Compound N-3 as a yellow solid compound.

The following target compound was synthesized in the same manner as in Preparative Example 1 above, except that intermediate A of Table 4 below was used instead of SM1 and intermediate B of Table 4 below was used instead of SM2, respectively.

**[Table 4]**

| Compound No. | Intermediate A | Intermediate B | Target Compound | Yield |
|---|---|---|---|---|
| N-2 | 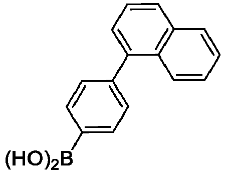 | 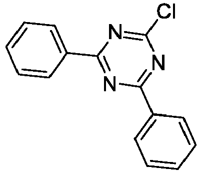 | 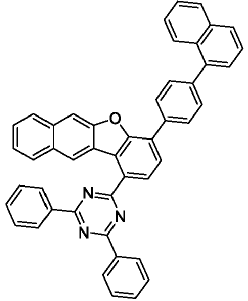 | 50% |
| N-3 | 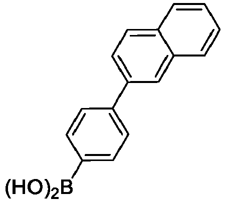 | 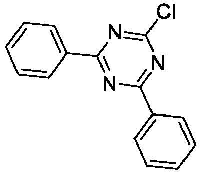 | 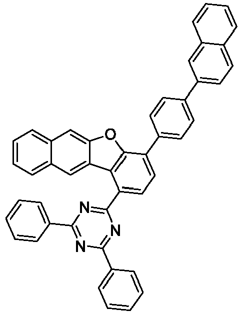 | 66% |
| N-6 | 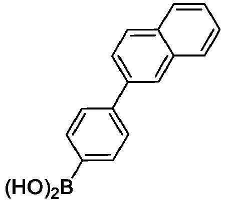 | 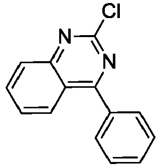 | 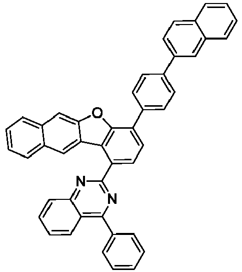 | 72% |
| N-7 | 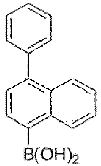 | 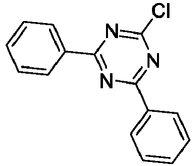 | 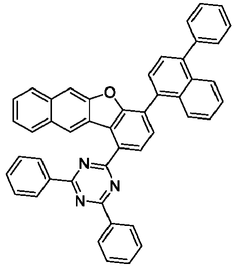 | 53% |

The remaining compounds represented by Formula 2 other than the compounds described in Preparative Example 2 and Table 4 above were also prepared in the same manner as in the preparative examples described above, and the synthesis results are shown in Tables 5 and 6 below.

**[Table 5]**

| Compound | FD-MS | Compound | FD-MS |
|---|---|---|---|
| N-1 | m/z=727.26 (C₅₃H₃₃N₃O=727.85) | N-5 | m/z=651.23 (C₄₇H₂₉N₃O=651.75) |
| N-9 | m/z=651.23 (C₄₇H₂₉N₃O=651.75) | N-13 | m/z=651.23 (C₄₇H₂₉N₃O=651.75) |
| N-17 | m/z=651.23 (C₄₇H₂₉N₃O=651.75) | N-21 | m/z=651.23 (C₄₇H₂₉N₃O=651.75) |
| N-25 | m/z=650.24 (C₄₈H₃ON₂O=650.76) | N-29 | m/z=651.23 (C₄₇H₂₉N₃O=651.75) |
| N-33 | m/z=651.23 (C₄₇H₂₉N₃O=651.75) | N-37 | m/z=651.23 (C₄₇H₂₉N₃O=651.75) |
| N-41 | m/z=727. 26 (C₅₃H₃₃N₃O=727.85) | N-45 | m/z=701. 25 (C₅₁H₃₁N₃O= 701.81) |
| N-49 | m/z=651.23 (C₄₇H₂₉N₃O=651.75) | N-53 | m/z=651.23 (C₄₇H₂₉N₃O=651.75) |
| N-57 | m/z=700.25 (C₅₂H₃₂N₂O=700.82) | N-61 | m/z=651.23 (C₄₇H₂₉N₃O=651.75) |
| N-65 | m/z=701. 25 (C₅₁H₃₁N₃O=701.81) | N-69 | m/z=717. 28 (C₅₂H₃₅N₃O=717.85) |
| N-73 | m/z=651.23 (C₄₇H₂₉N₃O=651.75) | N-77 | m/z=651.23 (C₄₇H₂₉N₃O=651.75) |

**[Table 6]**

| Compound | ¹H NMR(CDCl₃, 200Mz) |
|---|---|
| N-25 | δ= 8.55(2H, m), 8.23(1H, s), 8.16(2H, t), 8.01(2H, s), 7.79(6H, m), 7.67~7.63(4H, m), 7.51-7.41(13H, m). |
| N-45 | δ= 8.28(2H, d), 8.16(2H, d), 8.00~7.81(10H, m), 7.73-7.51(13H, m), 7.41(1H, t), 7.25(2H, d) |
| N-57 | δ= 8.30(2H, d), 8.16(3H, d), 8.00(2H, t), 7.92-7.83(6H, m), 7.73-7.41(15H, m), 7.25(4H, s) |
| N-65 | δ= 8.55-8.54(2H, m), 8.42(1H, d), 8.28(4H, d), 8.16-8.04(3H, m), 7.67-6.41(15H, m), 7.25(4H, d) |
| N-78 | δ= 8.55-8.54(2H, m), 8.42(1H, d), 8.28-8.24(3H, m), 8.16(1H, t), 8.08-8.04(2H, m), 7.70-7.41(20H, m). |

### <Experimental Example 1>

### (1) Manufacturing of organic light-emitting devices

A glass substrate coated with a thin film of indium tin oxide (ITO) to a thickness of 1500 Å was washed with distilled water ultrasonic waves. After washing with distilled water, it was ultrasonically washed with a solvent such as acetone, methanol, isopropyl alcohol, and the like, and dried, and then treated with ultraviolet ozone (UVO) for 5 minutes using UV in a UV cleaner. Thereafter, the substrate was transferred to a plasma cleaner (PT), and then plasma-treated in a vacuum to increase the work function of ITO and remove the residual film, and transferred to a thermal deposition equipment for organic deposition.

2-TNATA (4,4',4'-tris [2-naphthyl(phenyl)amino]triphenylamine) as a hole injection layer and NPB (N,N'-di(1-naphthyl)-N,N'-diphenyl-(1,1'-biphenyl)-4,4'-diamine) as a hole transport layer were formed on the ITO transparent electrode (anode), which are a common layer, respectively.

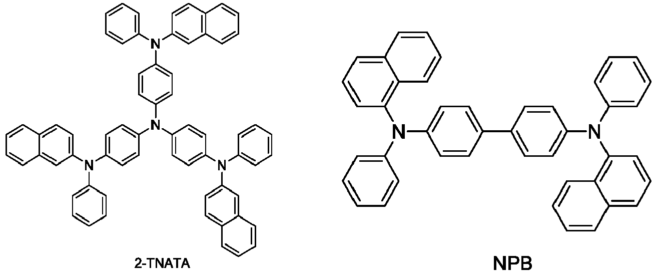

A light-emitting layer was thermally vacuum deposited thereon as follows. The light-emitting layer was formed by depositing a single or two kinds of compounds described in Table 7 below as a red host through one source, and doping (piq)₂(Ir) (acac) to the host at 3% using (piq)₂(Ir) (acac) as a red phosphorescent dopant and depositing it to a thickness of 500 Å. Thereafter, BCP was deposited to 60 Å as a hole-blocking layer, and Alq₃ was deposited to 200 Å thereon as an electron transport layer. Thereafter, bathocuproine (BCP) was deposited to a thickness of 60 Å as a hole-blocking layer, and Alq₃ was deposited to a thickness of 200 Å thereon as an electron transport layer. Finally, lithium fluoride (LiF) was deposited to a thickness of 10 Å on the electron transport layer to form an electron injection layer, and then an aluminum (Al) was deposited to a thickness of 1,200 Å on the electron injection layer to form a cathode, thereby manufacturing an organic light-emitting device.

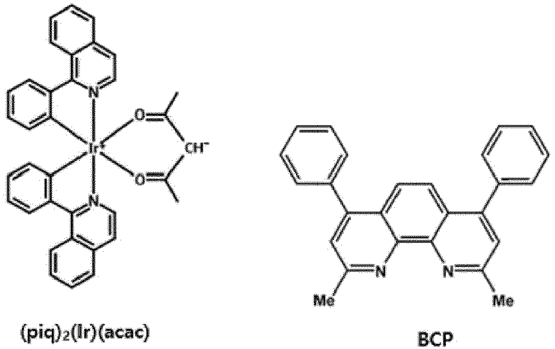

On the other hand, all organic compounds required for manufacturing OLED devices were purified by vacuum sublimation under 10⁻⁶ to 10⁻⁸ torr for each material, and used for manufacturing an organic light-emitting diode(OLED) device.

### (2) Driving voltage and luminous efficiency of organic light-emitting devices

For the organic light-emitting device manufactured as described above, electroluminescence (EL) properties were measured with M7000 from McScience Inc., and based on the measured results, T₉₀ was measured when the reference luminance was 6,000 cd/m² through the lifetime measuring device (M6000) manufactured by McScience Inc. The properties of the organic light-emitting device of the present invention are as shown in Table 7 below. In the above, T₉₀ means a lifetime (unit: time (h)) that is a time at which the luminance become 90% relative to the initial luminance.

The results of measuring the driving voltage, luminous efficiency, color coordinates (CIE), and lifetime of the organic light-emitting devices manufactured according to the present invention are as shown in Table 7 below. In addition, the results of measuring the driving voltage, luminous efficiency, color coordinates (CIE), and lifetime of the organic light-emitting devices manufactured using the following comparative compound as a compound for the light-emitting layer are as shown in Table 7 below.

**[Table 7]**

| | Compound no. | | Driving Voltage (V) | Efficiency (cd/A) | Color coordinates (x, Y) | lifetime (T₉₀) |
|---|---|---|---|---|---|---|
| Example 1 | 5 | | 3.79 | 40.2 | (0.682, 0.316) | 280 |
| Example 2 | 7 | | 3.52 | 43.7 | (0.683, 0.315) | 288 |
| Example 3 | 28 | | 3.87 | 41.3 | (0.681, 0.318) | 296 |
| Example 4 | 30 | | 3.79 | 40.9 | (0.680, 0.319) | 265 |
| Example 5 | 37 | | 3.81 | 45.5 | (0.680, 0.319) | 259 |
| Example 6 | 48 | | 3.68 | 46.7 | (0.682, 0.316) | 289 |
| Example 7 | 61 | | 3.91 | 36.9 | (0.683, 0.315) | 294 |
| Example 8 | 79 | | 4.14 | 29.0 | (0.681, 0.318) | 190 |
| Example 9 | 83 | | 4.06 | 27.9 | (0.680, 0.319) | 165 |
| Example 10 | 99 | | 4.53 | 23.2 | (0.681, 0.318) | 187 |
| Example 11 | 105 | | 4.69 | 26.8 | (0.682, 0.316) | 165 |
| Example 12 | 139 | | 4.98 | 30.8 | (0.683, 0.315) | 159 |
| Example 13 | 161 | | 3.09 | 43.5 | (0.681, 0.318) | 296 |
| Example 14 | 168 | | 3.03 | 44.9 | (0.682, 0.316) | 302 |
| Example 15 | 176 | | 3.18 | 42.7 | (0.683, 0.315) | 262 |
| Example 16 | 192 | | 3.59 | 43.1 | (0.681, 0.318) | 244 |
| Example 17 | 202 | | 3.24 | 39.8 | (0.680, 0.319) | 238 |
| Example 18 | 214 | | 3.41 | 43.5 | (0.680, 0.319) | 292 |
| Example 19 | 239 | | 3.88 | 41.4 | (0.680, 0.319) | 257 |
| Example 20 | 37:N-31 | 1:1 | 3.08 | 48.3 | (0.681, 0.318) | 310 |
| Example 21 | 37:N-2 | 1:1 | 2.98 | 49.5 | (0.680, 0.319) | 327 |
| Example 22 | 37:N-63 | 1:1 | 3.12 | 48.7 | (0.680, 0.319) | 318 |
| Example 23 | 52:N-2 | 3:1 | 3.46 | 49.38 | (0.681, 0.318) | 306 |
| Example 24 | | 1:1 | 3.52 | 51.02 | (0.681, 0.318) | 321 |
| Example 25 | | 1:3 | 3.69 | 48.69 | (0.681, 0.318) | 318 |
| Example 26 | 179 | | 3.88 | 36.5 | (0.683, 0.315) | 221 |
| Example 27 | 180 | | 3.90 | 33.8 | (0.681, 0.318) | 103 |
| Example 28 | 183 | | 3.98 | 31.2 | (0.680, 0.319) | 121 |
| Comparative Example 7 | A: N-2=1:1 | | 4.92 | 21.2 | (0.680, 0.319) | 81 |
| Comparative | B: N-63 =1:1 | | 5.01 | 19.9 | (0.681, 0.318) | 94 |
| Example 8 | | | | | | |
| Comparative Example 9 | C: N-31=1:1 | | 4.89 | 20.8 | (0.679, 0.320) | 103 |
| Example 29 | 167: N-63 | 7 : 1 | 3.49 | 48.9 | (0.682, 0.316) | 280 |
| Example 30 | | 5:1 | 3.49 | 49.8 | (0.681, 0.318) | 279 |
| Example 31 | | 3:1 | 3.52 | 48.5 | (0.681, 0.318) | 288 |
| Example 32 | | 1:1 | 3.60 | 46.3 | (0.682, 0.316) | 292 |
| Comparative Example 1 | A | | 5.43 | 8.3 | (0.685, 0.315) | 10 |
| Comparative Example 2 | B | | 5.36 | 8.7 | (0.686, 0.314) | 13 |
| Comparative Example 3 | C | | 5.29 | 11.2 | (0.687, 0.313) | 32 |
| Comparative Example 4 | D | | 5.91 | 9.5 | (0.679, 0.320) | 27 |
| Comparative Example 5 | E | | 5.58 | 18.3 | (0.682, 0.316) | 100 |
| Comparative Example 6 | F | | 4.95 | 21.8 | (0.681, 0.318) | 98 |

### <Comparative Compounds>

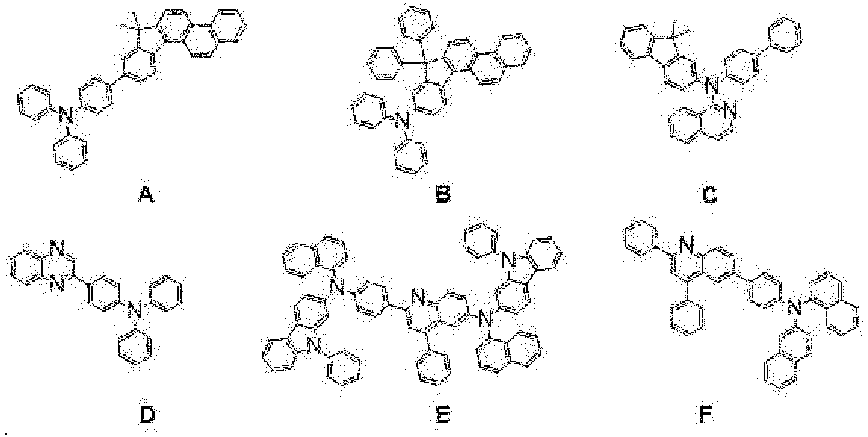

Looking at the results of Table 7 above, it could be confirmed that when the combination of the heterocyclic compound represented by Formula 1 (p-HOST) and the heterocyclic compound represented by Formula 2 (n-HOST) was used as a light-emitting layer of the organic light-emitting device, excellent effects were obtained in lifetime, luminous efficiency, and driving voltage properties compared to the case of using the heterocyclic compound represented by Formula 1 alone.

In particular, it could be confirmed that the heterocyclic compound represented by Formula 1 provides an appropriate energy level and thermal stability to the organic light-emitting device by substituting arylamines including a linking group on the benzene ring containing a nitrogen atom in the quinoline structure, and an organic light-emitting device with improved lifetime, driving stability and efficiency may be manufactured by using the compound represented by Formula 1.

It can be confirmed that when the compound represented by Formula 1 of the present invention (P-type) and the compound represented by Formula 2 of the present invention (N-type) are included at the same time, better efficiency and lifetime effects are obtained. From this, it can be expected that the exciplex phenomenon occurs when both compounds are included at the same time.

The exciplex phenomenon is a phenomenon in which energy having the size of the HOMO energy level of the donor (p-host) and the LUMO energy level of the acceptor (n-host) is emitted through electron exchange between two molecules. When the exciplex phenomenon occurs between two molecules, a reverse intersystem crossing (RISC) occurs, which may increase the internal quantum efficiency of fluorescence up to 100%. When a donor (p-host) with good hole transport ability and an acceptor (n-host) with good electron transport ability are used as a host for the light-emitting layer, since holes are injected into the p-host and electrons are injected into the n-host, the driving voltage may be lowered, which may help to improve lifetime.

In the present invention, it could be confirmed that when the compound represented by Formula 1 serving as a donor and the compound represented by Formula 2 serving as an acceptor were used as a host of the light-emitting layer, excellent device properties were obtained.

## Claims

1. A heterocyclic compound represented by following Formula 1: 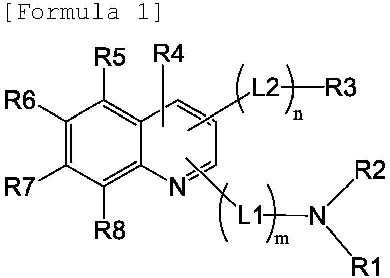 wherein,
L1 and L2 are the same as or different from each other and are each independently a single bond; a substituted or unsubstituted C6 to C60 arylene group; or a substituted or unsubstituted C2 to C60 heteroarylene group,
each of m and n is an integer from 0 to 5, with the proviso that when m is 2 or more, L1 is the same as or different from each other, and when n is 2 or more, L2 is the same as or different from each other,
R1 to R3 are the same as or different from each other and are each independently a substituted or unsubstituted C6 to C60 aryl group or a substituted or unsubstituted C2 to C60 heteroaryl group, and
R4 to R8 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; - SiR101R102R103; and -NR101R102, or two or more groups adjacent to each other combine with each other to form a substituted or unsubstituted C6 to C60 aromatic hydrocarbon ring or a substituted or unsubstituted C2 to C60 heterocycle, wherein R101, R102, and R103 are the same as or different from each other and are each independently a substituted or unsubstituted C1 to C60 alkyl group; a substituted or unsubstituted C6 to C60 aryl group; or a substituted or unsubstituted C2 to C60 heteroaryl group.

2. The heterocyclic compound according to claim 1, wherein Formula 1 is represented by any one of following Formulas 1-1 to 1-3: 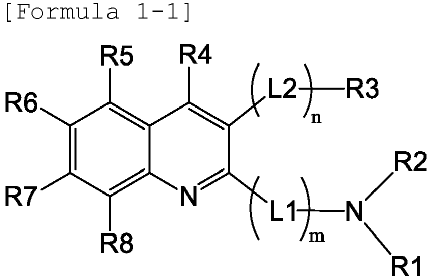 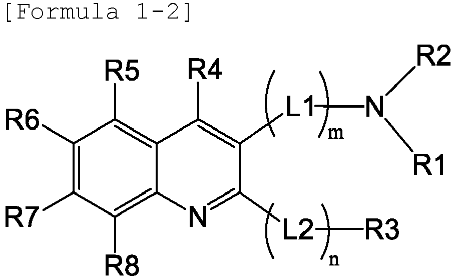 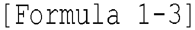 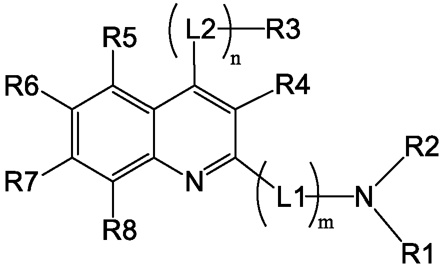 wherein,
L1, L2, R1 to R8, m, and n are the same as defined in Formula 1.

3. The heterocyclic compound according to claim 1, wherein L1 and L2 are the same as or different from each other and are each independently a single bond, a substituted or unsubstituted C6 to C60 arylene group, or a substituted or unsubstituted carbazolylene group.

4. The heterocyclic compound according to claim 1, wherein R4 to R8 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; - P(=O)R101R102; and -SiR101R102R103, or two or more groups adjacent to each other combine with each other to form a substituted or unsubstituted C6 to C60 aromatic hydrocarbon ring or a substituted or unsubstituted C2 to C60 heterocycle, wherein R101, R102, and R103 are the same as or different from each other and are each independently a substituted or unsubstituted C1 to C60 alkyl group; a substituted or unsubstituted C6 to C60 aryl group; or a substituted or unsubstituted C2 to C60 heteroaryl group.

5. The heterocyclic compound according to claim 1, wherein Formula 1 is represented by any one of the following compounds:

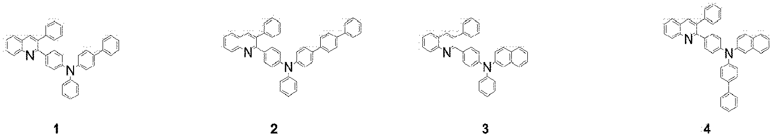

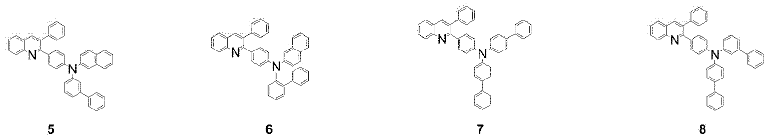

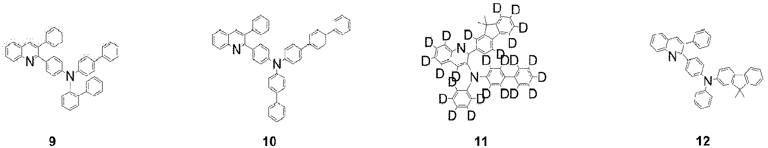

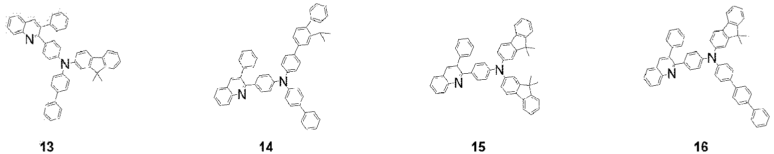

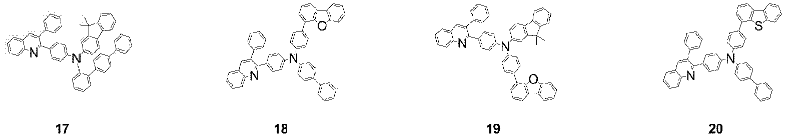

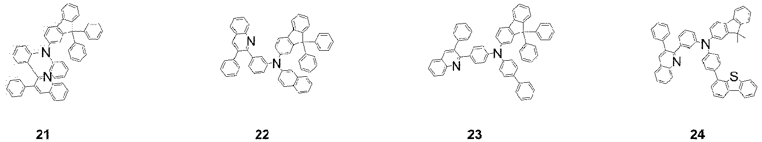

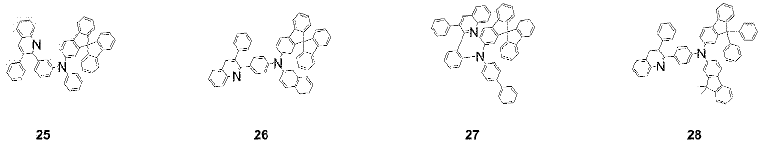

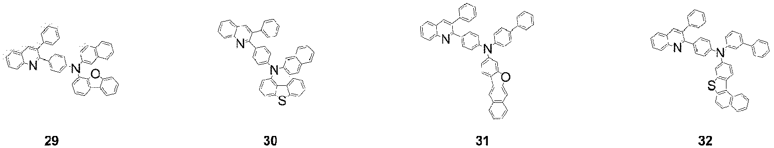

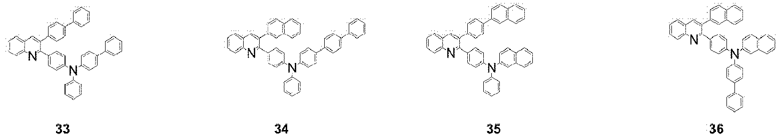

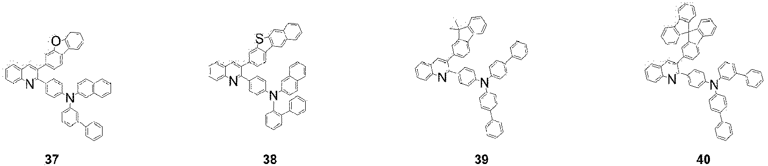

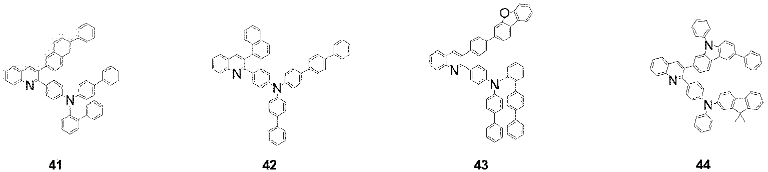

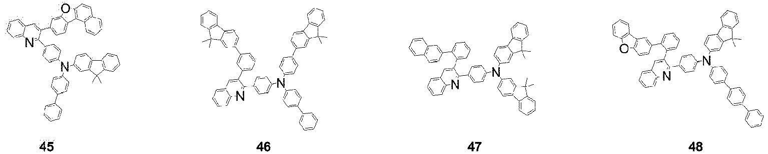

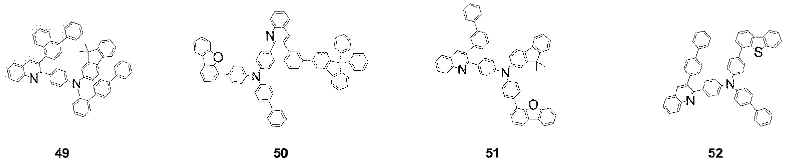

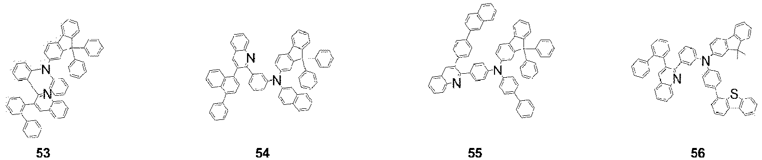

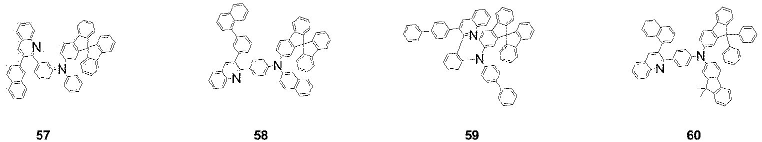

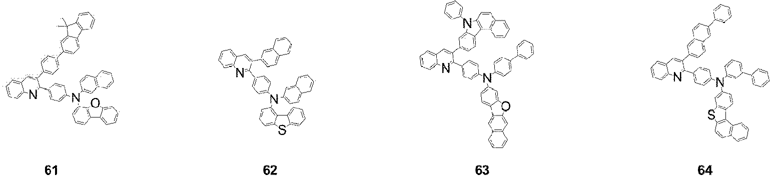

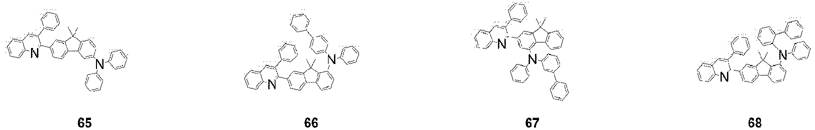

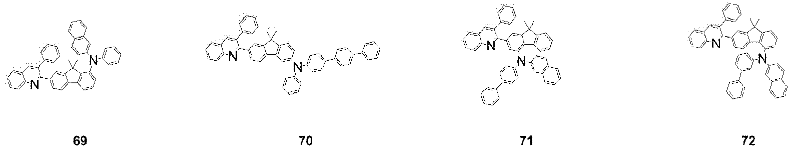

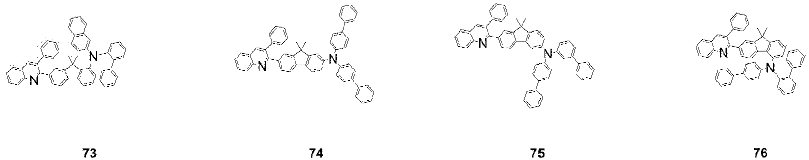

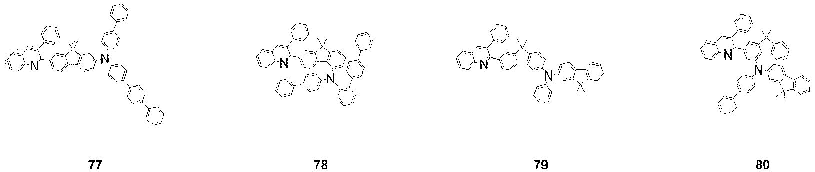

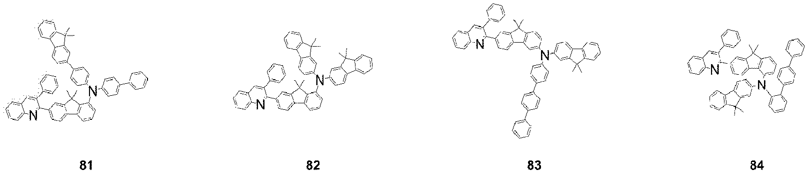

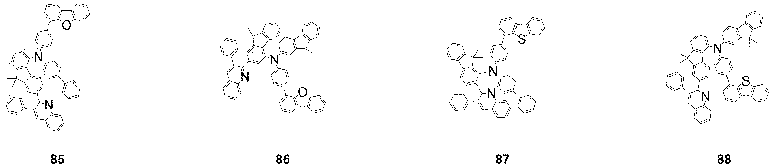

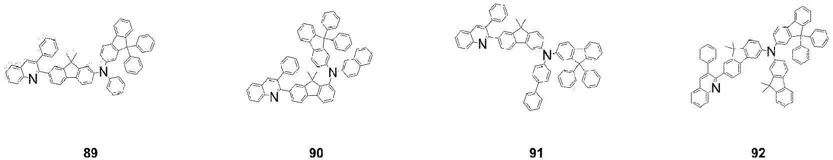

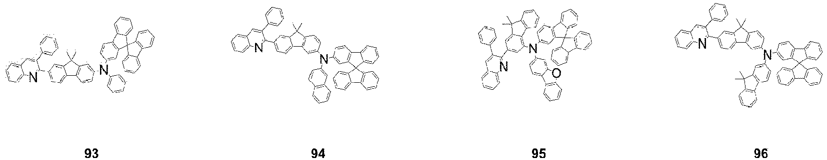

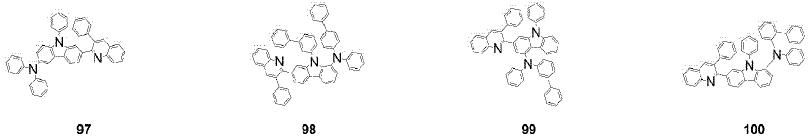

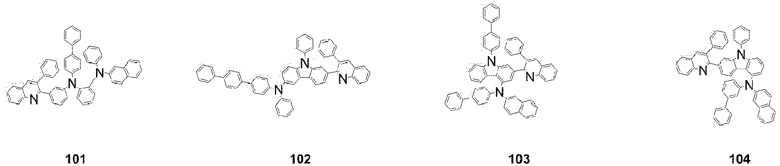

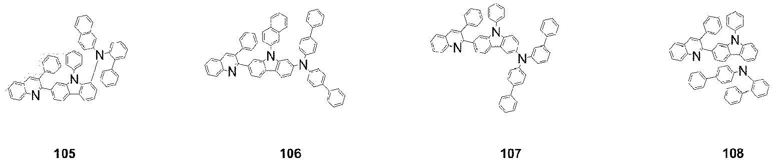

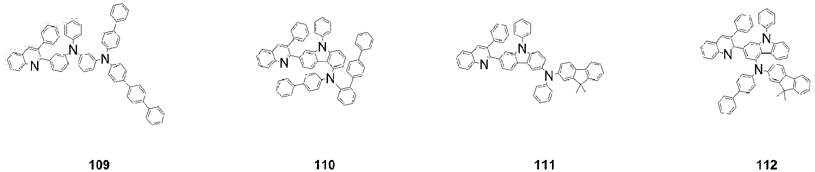

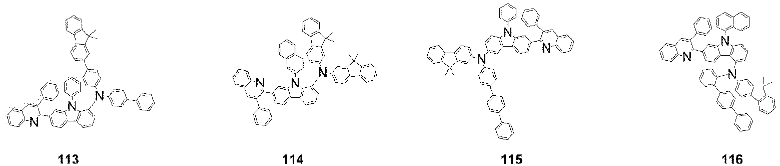

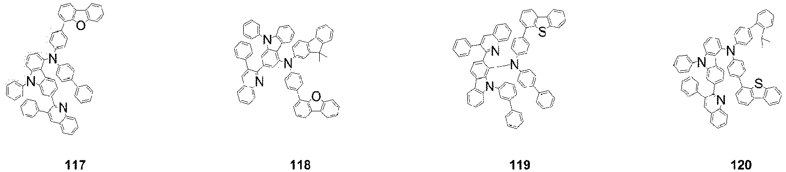

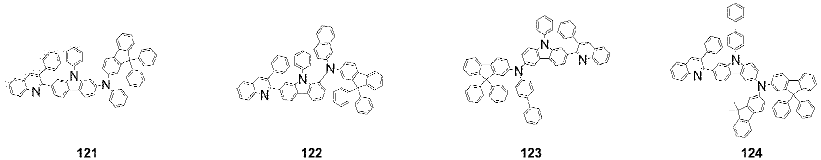

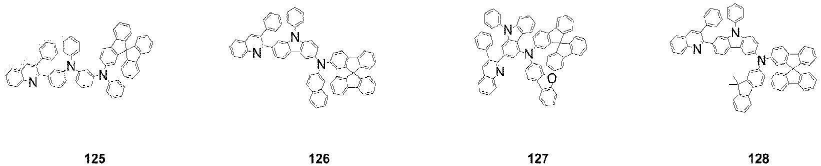

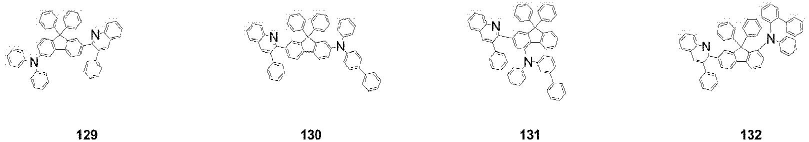

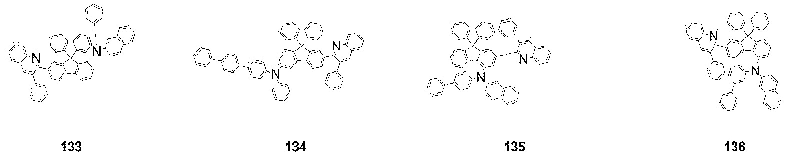

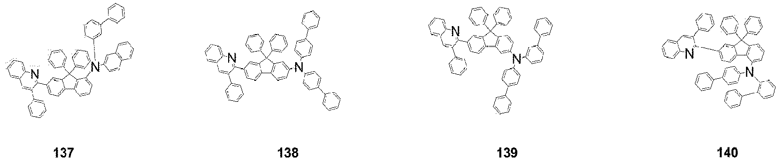

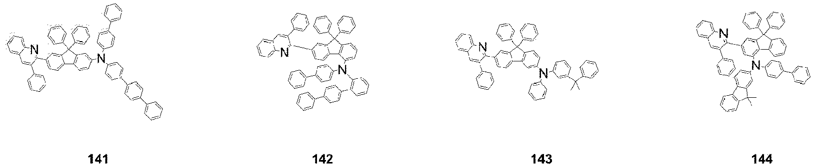

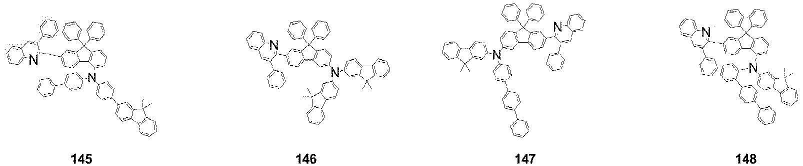

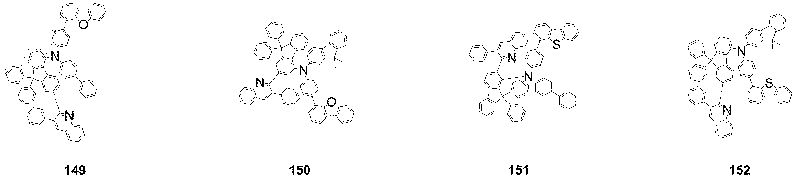

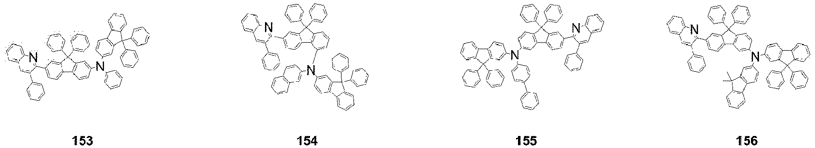

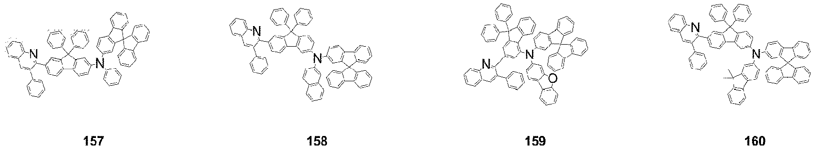

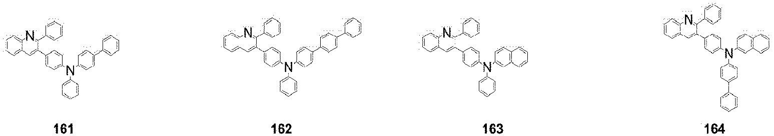

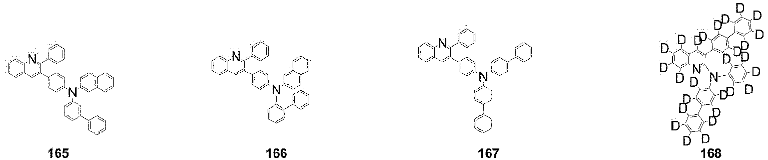

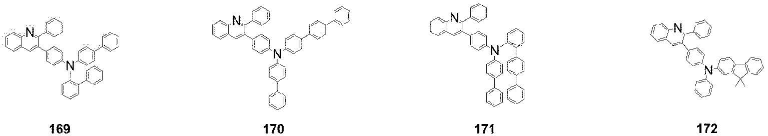

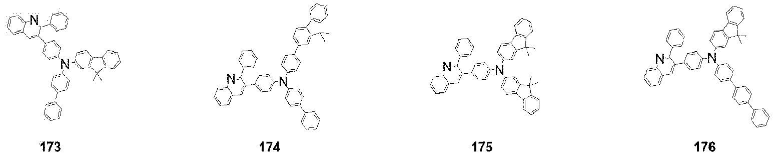

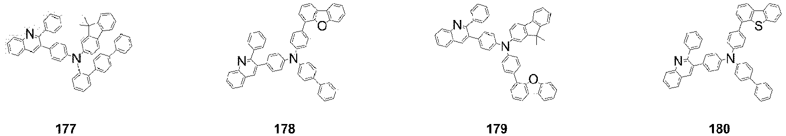

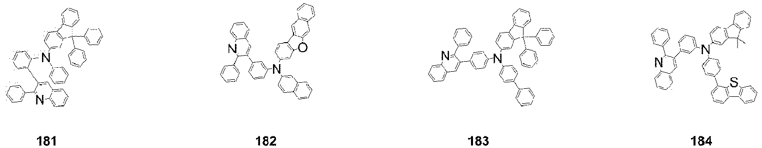

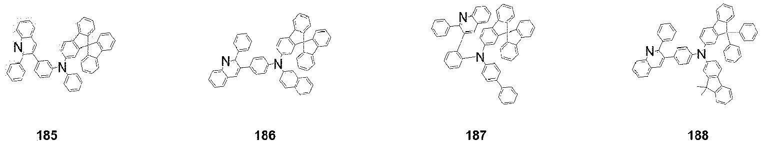

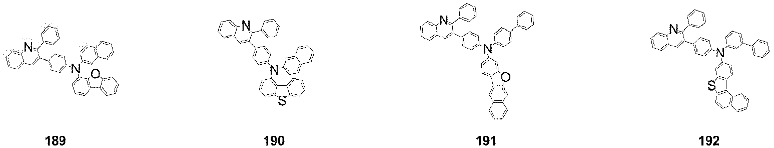

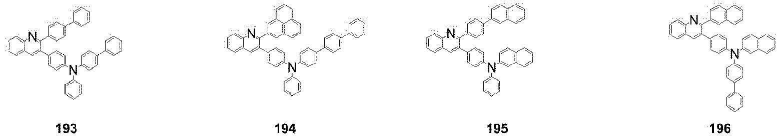

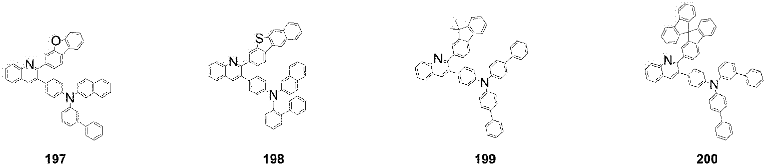

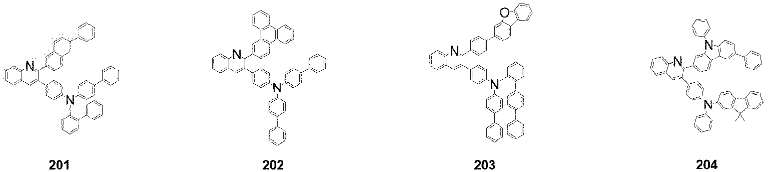

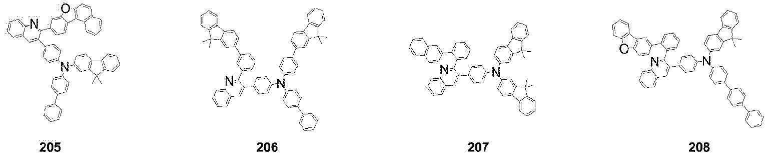

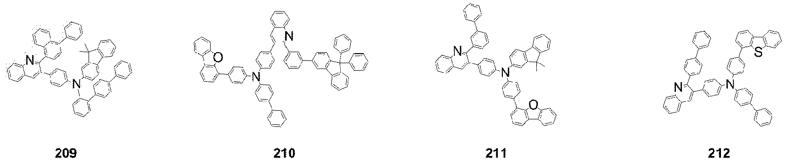

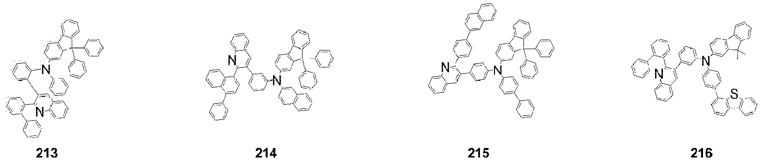

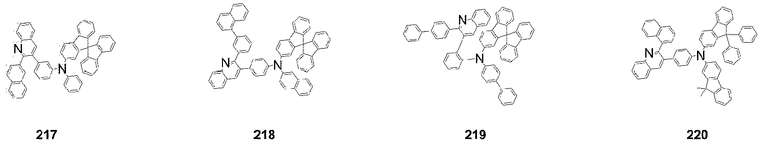

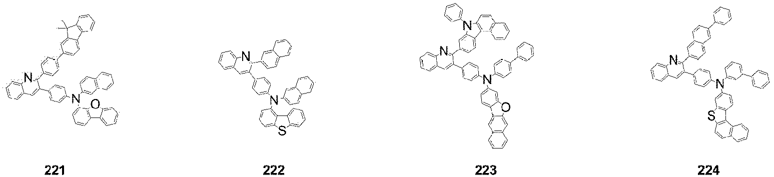

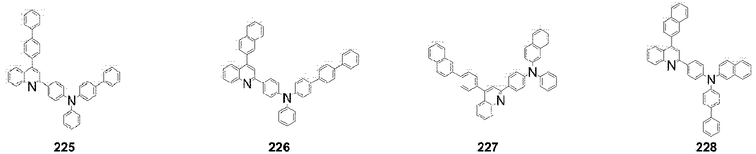

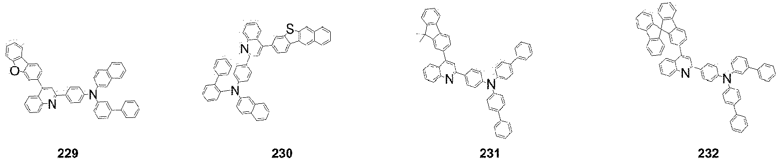

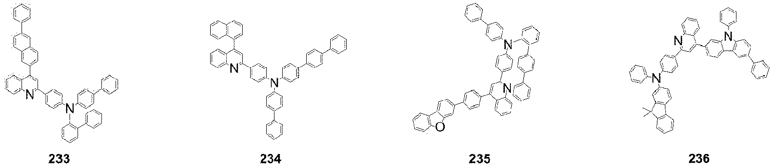

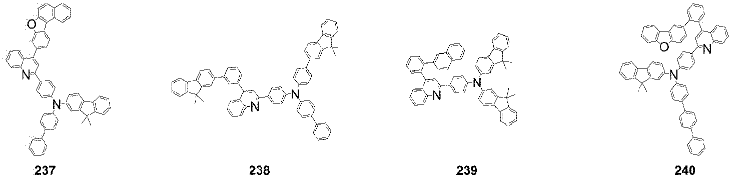

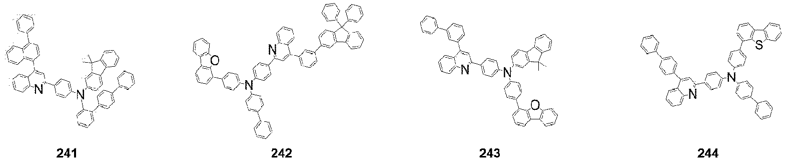

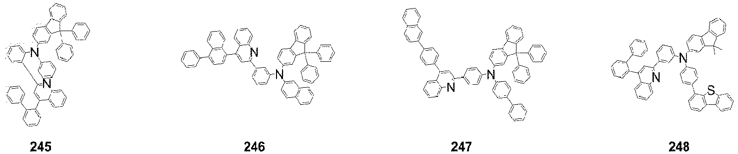

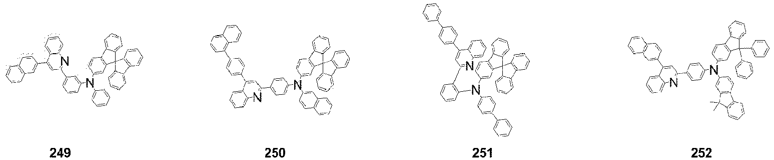

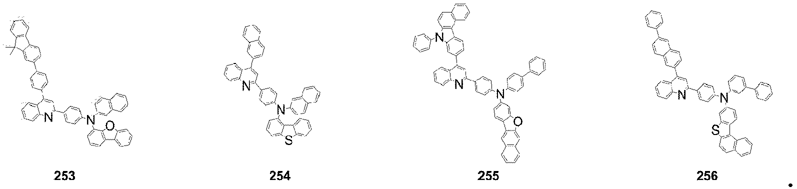

6. An organic light-emitting device comprising:
a first electrode;
a second electrode provided to face the first electrode; and
one or more organic layers provided between the first electrode and the second electrode, and
wherein at least one of the one or more of organic layers comprise the heterocyclic compound according to any one of claims 1 to 5.

7. The organic light-emitting device according to claim 6, wherein the organic layer further comprises a heterocyclic compound represented by following Formula 2: 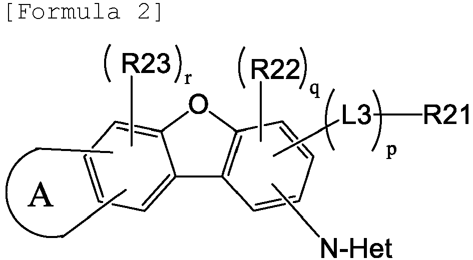 wherein,
N-Het is a substituted or unsubstituted, C2 to C60 monocyclic or polycyclic heterocyclic group containing one or more N,
L3 is a single bond; a substituted or unsubstituted C6 to C60 arylene group; or a substituted or unsubstituted C2 to C60 heteroarylene group, and p is an integer from 0 to 5, with the proviso that when p is 2 or more, L3 is the same as or different from each other,
A is a substituted or unsubstituted C6 to C60 aryl ring or a substituted or unsubstituted C2 to C60 heteroaryl ring,
R21 to R23 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R201R202; - SiR201R202R203; and -NR201R202, or two or more groups adjacent to each other combine with each other to form a substituted or unsubstituted C6 to C60 aromatic hydrocarbon ring or a substituted or unsubstituted C2 to C60 heterocycle, wherein R201, R202, and R203 are the same as or different from each other and are each independently a substituted or unsubstituted C1 to C60 alkyl group; a substituted or unsubstituted C6 to C60 aryl group; or a substituted or unsubstituted C2 to C60 heteroaryl group, and
each of q and r is an integer from 0 to 2, with the proviso that when q is 2, R22 is the same as or different from each other, and when r is 2, R23 is the same as or different from each other.

8. The organic light-emitting device according to claim 7, wherein Formula 2 is a heterocyclic compound represented by any one of following Formulas 2-1 to 2-3: 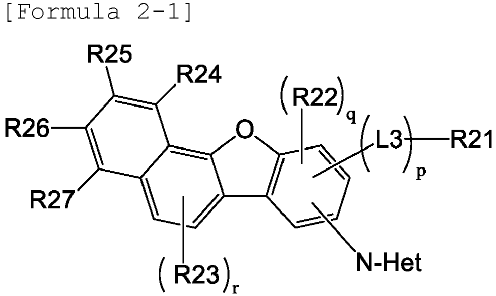 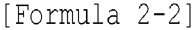 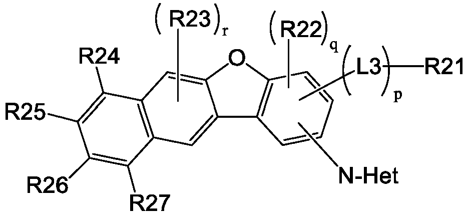 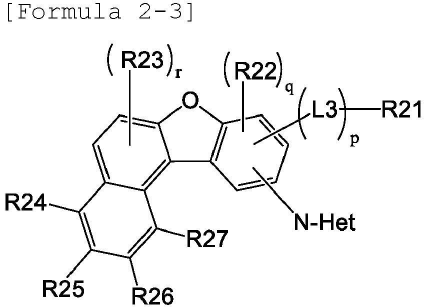 wherein,
R24 to R27 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R201R202; - SiR201R202R203; and -NR201R202, or two or more groups adjacent to each other combine with each other to form a substituted or unsubstituted C6 to C60 aromatic hydrocarbon ring or a substituted or unsubstituted C2 to C60 heterocycle, wherein R201, R202, and R203 are the same as or different from each other and are each independently a substituted or unsubstituted C1 to C60 alkyl group; a substituted or unsubstituted C6 to C60 aryl group; or a substituted or unsubstituted C2 to C60 heteroaryl group, and
N-Het, L3, R21 to R23, p, q, and r are the same as defined in Formula 2.

9. The organic light-emitting device according to claim 7, wherein N-Het is a heterocyclic compound represented by any one of following Formulas 3-1 to 3-4: 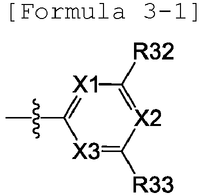 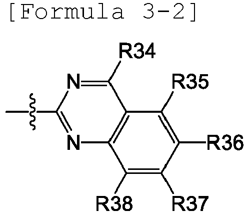 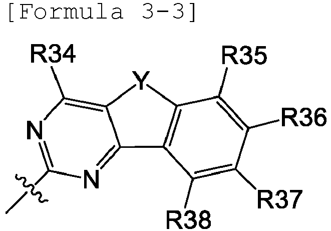 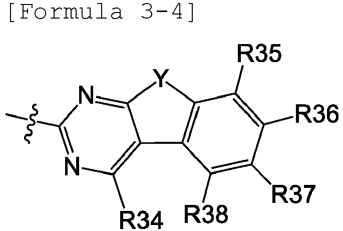 wherein,
X1 to X3 are the same as or different from each other and are each independently N or CR31, and at least two of X1 to X3 are N,
Y is O or S,
R32 to R34 are the same as or different from each other and are each independently a substituted or unsubstituted C6 to C60 aryl group or a substituted or unsubstituted C2 to C60 heteroaryl group, and
R31 and R35 to R38 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R301R302; - SiR301R302R303; and -NR301R302, or two or more groups adjacent to each other combine with each other to form a substituted or unsubstituted C6 to C60 aromatic hydrocarbon ring or a substituted or unsubstituted C2 to C60 heterocycle, wherein R301, R302, and R303 are the same as or different from each other and are each independently a substituted or unsubstituted C1 to C60 alkyl group; a substituted or unsubstituted C6 to C60 aryl group; or a substituted or unsubstituted C2 to C60 heteroaryl group.

10. The organic light-emitting device according to claim 7, wherein the heterocyclic compound represented by Formula 2 is any one selected from the following compounds:

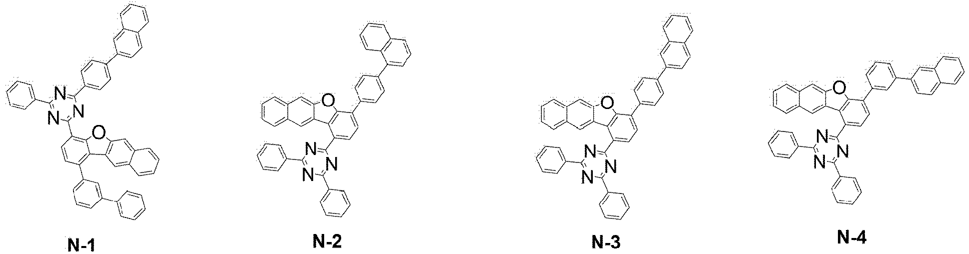

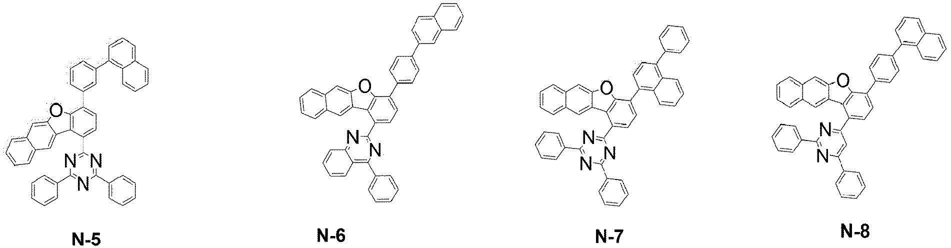

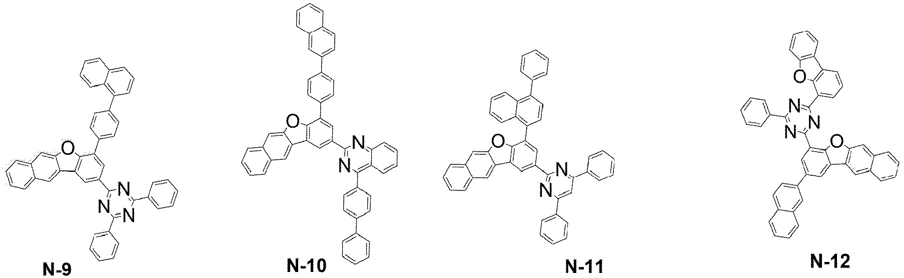

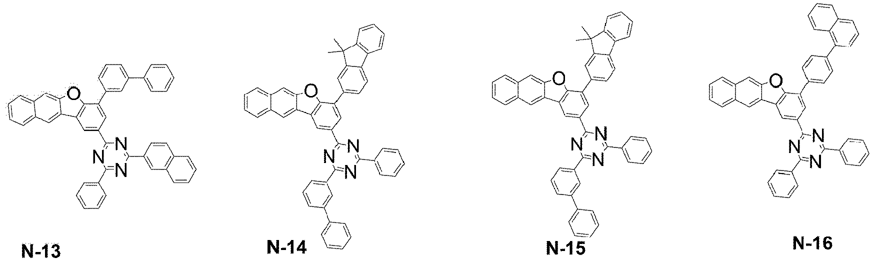

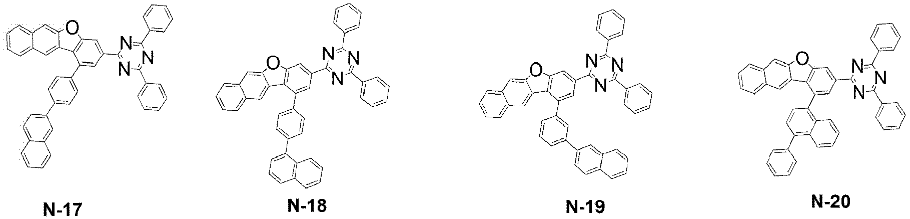

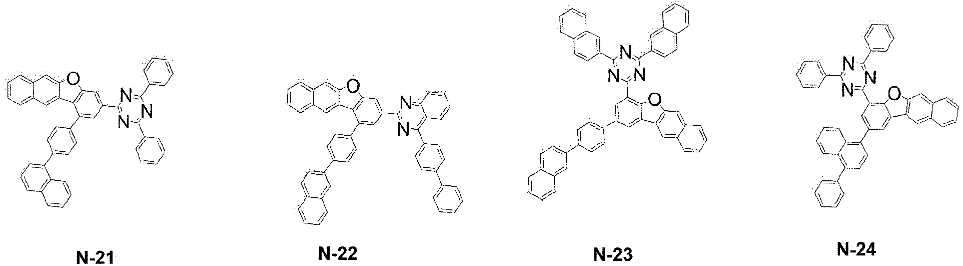

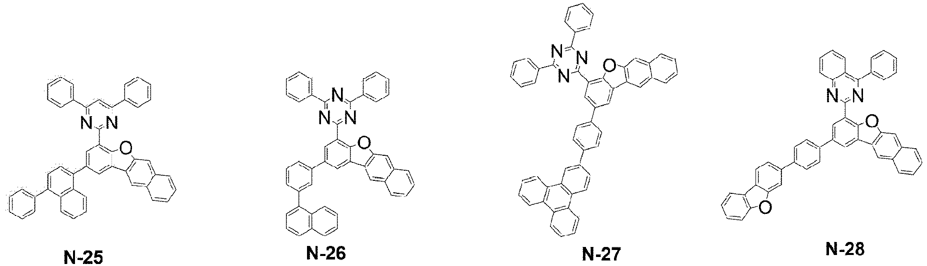

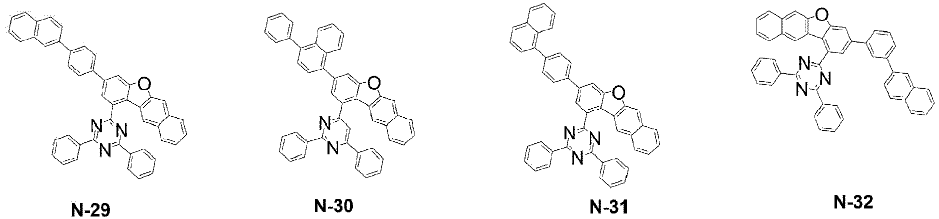

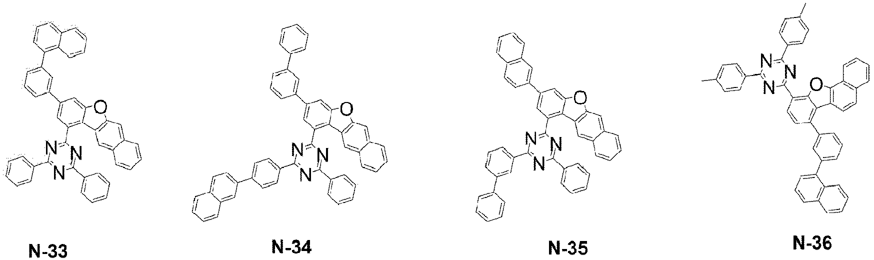

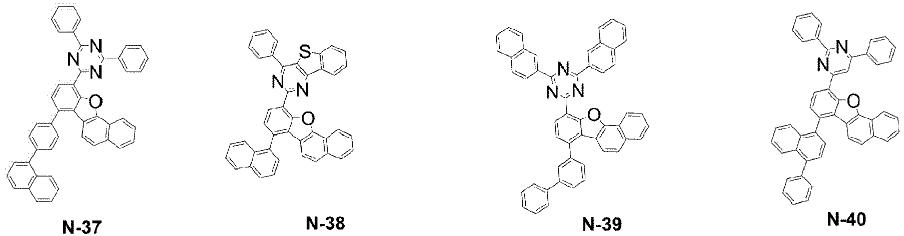

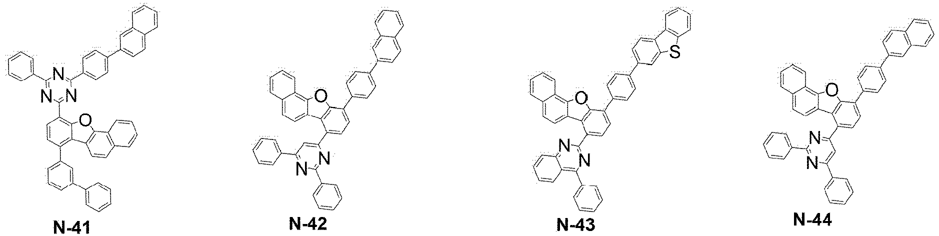

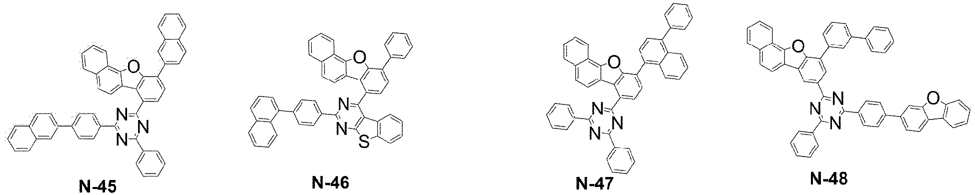

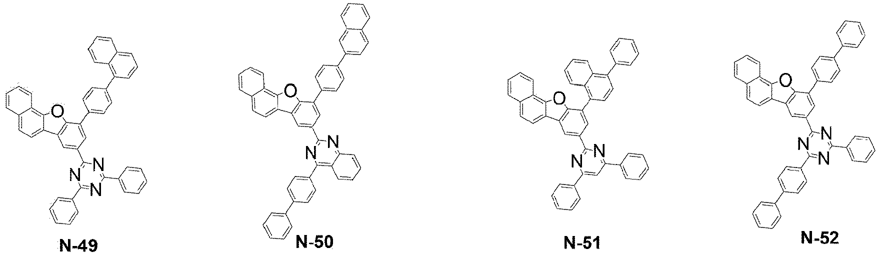

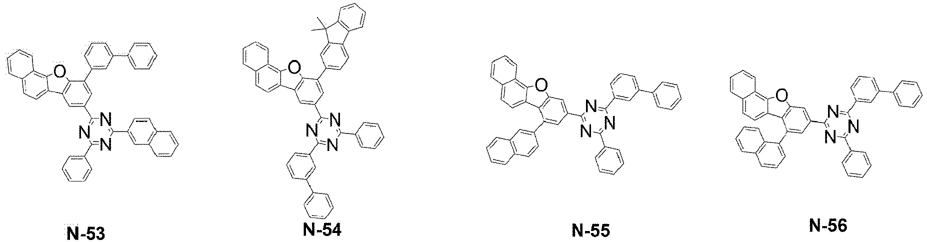

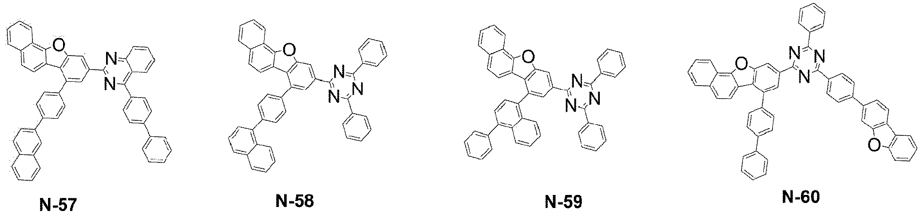

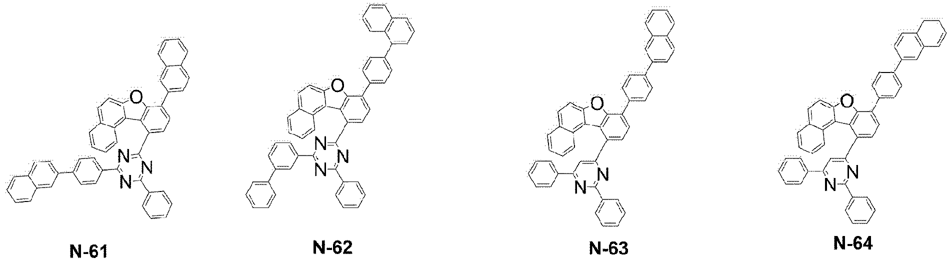

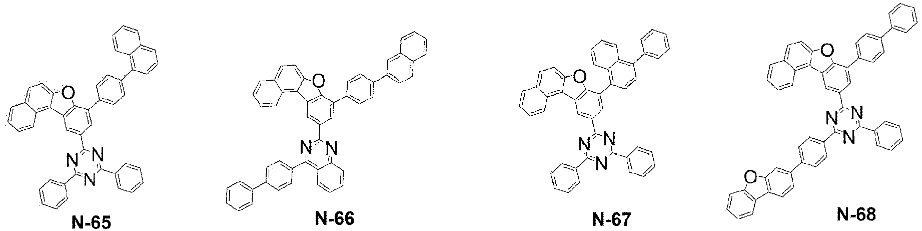

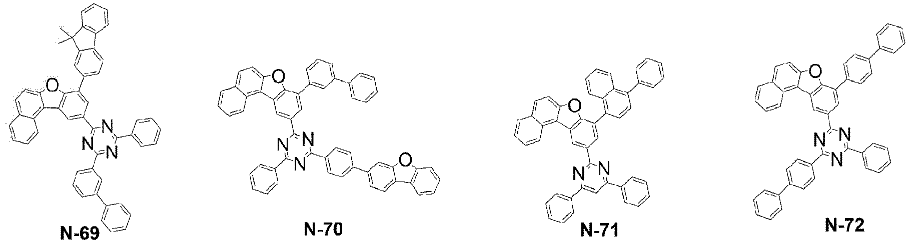

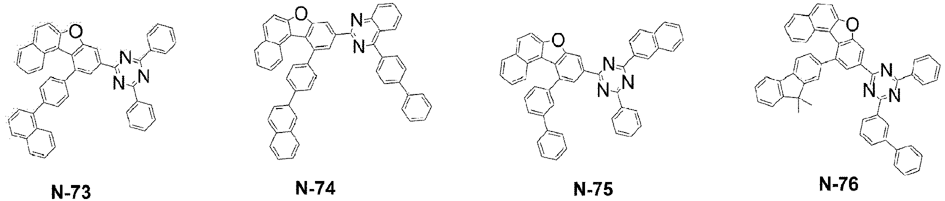

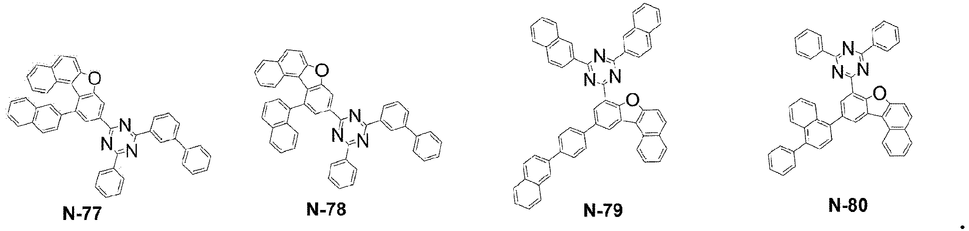

11. The organic light-emitting device according to claim 6, wherein the organic layer comprises a light-emitting layer, wherein the light-emitting layer comprises a host material, wherein the host material comprises the heterocyclic compound according to any one of claims 1 to 5 and a heterocyclic compound represented by following Formula 2: 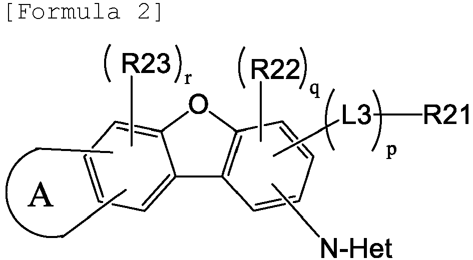 wherein,
N-Het is a substituted or unsubstituted, C2 to C60 monocyclic or polycyclic heterocyclic group containing one or more N,
L3 is a single bond; a substituted or unsubstituted C6 to C60 arylene group; or a substituted or unsubstituted C2 to C60 heteroarylene group; and p is an integer from 0 to 3, with the proviso that when p is 2 or more, L3 is the same as or different from each other,
A is a substituted or unsubstituted C6 to C60 aryl ring or a substituted or unsubstituted C2 to C60 heteroaryl ring,
R21 to R23 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R201R202; - SiR201R202R203; and -NR201R202, or two or more groups adjacent to each other combine with each other to form a substituted or unsubstituted C6 to C60 aromatic hydrocarbon ring or a substituted or unsubstituted C2 to C60 heterocycle, wherein R201, R202, and R203 are the same as or different from each other and are each independently a substituted or unsubstituted C1 to C60 alkyl group; a substituted or unsubstituted C6 to C60 aryl group; or a substituted or unsubstituted C2 to C60 heteroaryl group, and
each of q and r is an integer from 0 to 2, with the proviso that when q is 2, R22 is the same as or different from each other, and when r is 2, R23 is the same as or different from each other.

12. The organic light-emitting device according to claim 6, wherein the organic light-emitting device further comprises one or two or more layers selected from the group consisting of a light-emitting layer, a hole injection layer, a hole transport layer, an electron injection layer, an electron transport layer, an electron-blocking layer, and a hole-blocking layer.

13. A composition for an organic layer of an organic light-emitting device, comprising the heterocyclic compound according to any one of claims 1 to 5 and a heterocyclic compound represented by following Formula 2: 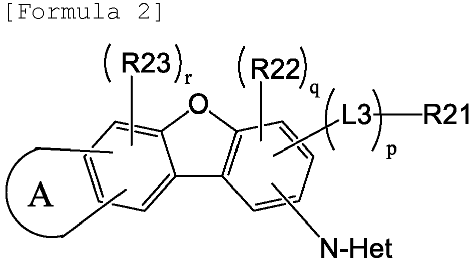 wherein,
N-Het is a substituted or unsubstituted, C2 to C60 monocyclic or polycyclic heterocyclic group containing one or more N,
L3 is a single bond; a substituted or unsubstituted C6 to C60 arylene group; or a substituted or unsubstituted C2 to C60 heteroarylene group, and p is an integer from 0 to 3, with the proviso that when p is 2 or more, L3 is the same as or different from each other,
A is a substituted or unsubstituted C6 to C60 aryl ring or a substituted or unsubstituted C2 to C60 heteroaryl ring,
R21 to R23 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R201R202; - SiR201R202R203; and -NR201R202, or two or more groups adjacent to each other combine with each other to form a substituted or unsubstituted C6 to C60 aromatic hydrocarbon ring or a substituted or unsubstituted C2 to C60 heterocycle, wherein R201, R202, and R203 are the same as or different from each other and are each independently a substituted or unsubstituted C1 to C60 alkyl group; a substituted or unsubstituted C6 to C60 aryl group; or a substituted or unsubstituted C2 to C60 heteroaryl group, and
each of q and r is an integer from 0 to 2, with the proviso that when q is 2, R22 is the same as or different from each other, and when r is 2, R23 is the same as or different from each other.

14. The composition for an organic layer of an organic light-emitting device according to claim 13, wherein the weight ratio of the heterocyclic compound according to any one of claims 1 to 5 and the heterocyclic compound represented by Formula 2 is 1:10 to 10:1.

15. A method of manufacturing an organic light-emitting device, comprising the steps of:
preparing a substrate;
forming a first electrode on the substrate;
forming one or more organic layers on the first electrode; and
forming a second electrode on the one or more organic layers, and
wherein the step of forming the one or more organic layers comprises a step of forming the one or more organic layers using the composition for an organic layer of an organic light-emitting device according to claim 13.

16. The method of manufacturing an organic light-emitting device according to claim 15, wherein the step of forming the one or more organic layers comprises pre-mixing the heterocyclic compound represented by Formula 1 and the heterocyclic compound represented by Formula 2, and forming the one or more organic layers using a thermal vacuum deposition method.
